# EUROPEAN PATENT APPLICATION

(11) **EP 2 270 895 A2**
(43) Date of publication of application: **05.01.2011**
(21) Application number: 10181674.2
(22) Date of filing: 29.11.2000
(51) Int. Cl.: H01L 51/30, C09K 11/06, H05B 33/14

(54) **Complexes for OLEDs**

(30) Priority: 01.12.1999 US 452346
(62) Divisional of application: 08003327.7
(71) Applicant: The Trustees of Princeton University, Princeton NJ 08544-0036 (US); The University of Southern California, Los Angeles, CA 90007-4344 (US)
(72) Inventor: Thompson, Mark E., Anaheim, CA 92807 (US); Djurovich, Peter, Long Beach, CA 90007 (US); Lamansky, Sergey, Apple Valley, MN 55214 (US); Murphy, Drew, New Haven, CT 06511 (US); Kwong, Raymond, Plainsboro, NJ 08536 (US); Abdel-Razzaq, Feras, Los Angeles, CA 90089 (US); Forrest, Stephen R., Ann Arbor, MI 48109 (US); Baldo, Marc A., Cambridge, MA 02142 (US); Burrows, Paul, Kennewick, WA 99338 (US)
(74) Representative: Hansen, Norbert

(57) **Abstract**

A complex of formula LL'L"M, LL'MX, LMXX', or L₃M, wherein L, L', L", X, and X' are inequivalent, bidentate ligands, M is a metal that forms octahedral complexes, and L, L', and L" are monoanionic bidentate ligands coordinated to M through an sp² hybridized carbon and a heteroatom.

## Description

### I. FIELD OF INVENTION

The present invention relates to organometallic compounds of formula L₂MX, wherein L and X are distinct bidentate ligands, and M is a metal, especially iridium, their synthesis and use as dopants in certain hosts to form an emitting layer in organic light emitting devices.

### II. BACKGROUND OF THE INVENTION

### II. A. General Background

Organic light emitting devices (OLEDs) are comprised of several organic layers in which one of the layers is comprised of an organic material that can be made to electroluminesce by applying a voltage across the device, C. W. Tang ct al., Appl. Phys. Lett. 1987, 51, 913. Certain OLEDs have been shown to have sufficient brightness, range of color and operating lifetimes for use as a practical alternative technology to LCD-based full color flat-panel displays (S.R. Forrest, P.E. Burrows and M.E. Thompson, Laser Focus World, Feb. 1995). Since many of the thin organic films used in such devices are transparent in the visible spectral region, they allow for the realization of a completely new type of display pixel in which red (R), green (G), and blue (B) emitting OLEDs are placed in a vertically stacked geometry to provide a simple fabrication process, a small R-G-B pixel size, and a large fill factor, International Patent Application No. PCT/US95/15790.

A transparent OLED (TOLED), which represents a significant step toward realizing high resolution, independently addressable stacked R-G-B pixels, was reported in International Patent Application No. PCT/US97/02681 in which the TOLED had greater than 71% transparency when turned off and emitted light from both top and bottom device surfaces with high efficiency (approaching 1% quantum efficiency) when the device was turned on. The TOLED used transparent indium tin oxide (ITO) as the hole-injecting electrode and a Mg-Ag-ITO electrode layer for electron-injection. A device was disclosed in which the ITO side of the Mg-Ag-ITO layer was used as a hole-injecting contact for a second, different color-emitting OLED stacked on top of the TOLED. Each layer in the stacked OLED (SOLED) was independently addressable and emitted its own characteristic color. This colored emission could be transmitted through the adjacently stacked, transparent, independently addressable, organic layer or layers, the transparent contacts and the glass substrate, thus allowing the device to emit any color that could be produced by varying the relative output of the red and blue color-emitting layers.

PCT/US95/15790 application disclosed an integrated SOLED for which both intensity and color could be independently varied and controlled with external power supplies in a color tunable display device. The PCT/US95/15790 application, thus, illustrates a principle for achieving integrated, full color pixels that provide high image resolution, which is made possible by the compact pixel size. Furthermore, relatively low cost fabrication techniques, as compared with prior art methods, may be utilized for making such devices.

### II.B. Background of emission

### II.B.1. Basics

### II.B.1.a. Singlet and Triplet Excitons

Because light is generated in organic materials from the decay of molecular excited states or excitons, understanding their properties and interactions is crucial to the design of efficient light emitting devices currently of significant interest due to their potential uses in displays, lasers, and other illumination applications. For example, if the symmetry of an exciton is different from that of the ground state, then the radiative relaxation of the exciton is disallowed and luminescence will be slow and inefficient. Because the ground state is usually anti-symmetric under exchange of spins of electrons comprising the exciton, the decay of a symmetric exciton breaks symmetry. Such excitons are known as triplets, the term reflecting the degeneracy of the state. For every three triplet excitons that are formed by electrical excitation in an OLED, only one symmetric state (or singlet) exciton is created. (M.A. Baldo, D. F. O'Brien, M.E. Thompson and S. R. Forrest, Very high-efficiency green organic light-emitting devices based on electrophosphorescence, Applied Physics Letters, 1999, 75, 4-6.) Luminescence from a symmetry-disallowed process is known as phosphorescence. Characteristically, phosphorescence may persist for up to several seconds after excitation due to the low probability of the transition. In contrast, fluorescence originates in the rapid decay of a singlet exciton. Since this process occurs between states of like symmetry, it may be very efficient.

Many organic materials exhibit fluorescence from singlet excitons. However, only a very few have been identified which are also capable of efficient room temperature phosphorescence from triplets. Thus, in most fluorescent dyes, the energy contained in the triplet states is wasted. However, if the triplet excited state is perturbed, for example, through spin-orbit coupling (typically introduced by the presence of a heavy metal atom), then efficient phosphoresence is more likely. In this case, the triplet exciton assumes some singlet character and it has a higher probability of radiative decay to the ground state. Indeed, phosphorescent dyes with these properties have demonstrated high efficiency electroluminescence.

Only a few organic materials have been identified which show efficient room temperature phosphorescence from triplets. In contrast, many fluorescent dyes are known (C.H. Chen, J. Shi, and C.W. Tang, "Recent developments in molecular organic electroluminescent materials," Macromolecular Symposia, 1997, 125, 1-48; U. Brackmann, Lambdachrome Laser Dyes (Lambda Physik, Gottingen, 1997) and fluorescent efficiencies in solution approaching 100% are not uncommon. (C.H. Chen, 1997, op. cit.) Fluorescence is also not affected by triplet-triplet annihilation, which degrades phosphorescent emission at high excitation densities. (M. A. Baldo, et al., "High efficiency phosphorescent emission from organic electroluminescent devices," Nature, 1998, 395, 151-154; M. A. Baldo, M. E. Thompson, and S.R. Forrest, "An analytic model of triplet-triplet annihilation in electrophosphorescent devices," 1999). Consequently, fluorescent materials are suited to many electroluminescent applications, particularly passive matrix displays.

### II.B.1.b. Overview of invention relative to basics

This invention relates to complexes of formula L L' L" M, wherein L, L', and L" are distinct bidentate ligands and M is a metal of atomic number greater than 40 which forms octahedral complexes and is preferably a member of the third row (of the transition series of the periodic table) transition metals. Alternatively, M can be a member of the second row transition metals, or of the main group metals, such as Zr and Sb. Some of such organometallic complexes electroluminesce, with emission coming from the lowest energy ligand or MLCT state. Such electroluminescent compounds can be used as dopants in a host layer of an emitter layer in light emitting diodes. This invention is further directed to complexes of formula L L' L" M, wherein L, L', and L" are the same or different, wherein L, L', and L" are bidentate, monoanionic ligands, wherein M is a metal which forms octahedral complexes, is preferably a member of the third row of transition metals, more preferably Ir or Pt, and wherein the coordinating atoms of the ligands comprise sp² hybridized carbon and a heteroatom. The invention is further directed to L₂MX, wherein L and X are distinct bidentate ligands, wherein L coordinates to M via atoms of L comprising sp² hybridized carbon and heteroatoms, and wherein M is a metal forming an octahedral complex, preferably iridium (1r). These compounds can serve as dopants in a host layer which functions as a emitter layer in organic light emitting diodes.

The compounds of this invention can be made by the direct reaction of chloride bridged dimers of the form

L₂M(µ-Cl)₂ML₂

, wherein L a bidentate ligand, and M a metal such as Ir, with species XH which serve to introduce a bidentate ligand X. XH can be, for example, acetylacetone, 2-picolinic acid, or N-methylsalicyclanilide, and H stands for hydrogen. The resultant product is of formula L₂MX, wherein one can have an octahedral disposition of the bidentate ligands L, L, and X about M.

The resultant compounds of formula L₂MX can be used as phosphorescent emitters in organic light emitting devices. For example, the compound wherein L=(2-phenylbenzothiazole), X=acetylacetonate, and M=Ir (the compound abbreviated as BTIr) when used as a dopant in 4,4'-N,N'-dicarbazole-biphenyl (CBP)(at a level 12% by mass) to form an emitter layer in an OLED shows a quantum efficiency of 12%. For reference, the formula CBP is

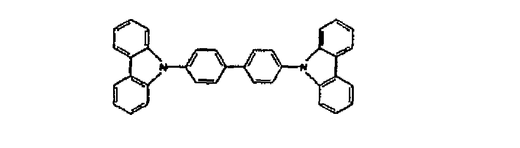

The synthetic process to make L₂MX may be used advantageously in a situation in which L, by itself, is fluorescent but the resultant L₂MX is phosphorescent. One specific example of this is where L=coumarin-6.

The synthetic process facilitates the combination of L and X pairs of certain desirable characteristics.

The appropriate selection of L and X allows color tuning of the complex L₂MX relative to L₃M. For example, Ir(ppy)₃ and (ppy)₂Ir(acac) both give strong green emission with a λₘₐₓ of 510 nm [ppy denotes phenyl pyridine]. However, if the X ligand is formed from picolinic acid instead of from acetylacetone, there is a small blue shift of about 15 nm.

Furthermore, X can be selected such that it has a certain HOMO level relative to the L₃M complex so that carriers (holes or electrons) might be trapped on X (or on L) without a deterioration of emission quality. In this way, carriers (holes or electrons) which might otherwise contribute to deleterious oxidation or reduction of the phosphor would be impeded. The carrier that is remotely trapped could readily recombine with the opposite carrier either intramolecularly or with the carrier from an adjacent molecule.

The invention, and its various embodiments, are discussed in more detail in the examples below. However the embodiments may operate by different mechanisms. Without limitation and without limiting the scope of the invention, we discuss the different mechanisms by which various embodiments of the invention may operate.

### II.B.1.c. Dexter and Förster mechanisms

To understand the different embodiments of this invention it is useful to discuss the underlying mechanistic theory of energy transfer. There are two mechanisms commonly discussed for the transfer of energy to an acceptor molecule. In the first mechanism of Dexter transport (D.L. Dexter, "A theory of sensitized luminescence in solids," J. Chem. Phys., 1953, 21, 836-850), the exciton may hop directly from one molecule to the next. This is a short-range process dependent on the overlap of molecular orbitals of neighboring molecules. It also preserves the symmetry of the donor and acceptor pair (E. Wigner and E.W. Wittmer, Uber die Struktur der zweiatomigen Molekelspektren nach der Quantenmechanik, Zeitschrift fur Physik. 1928, 51, 859-886; M. Klessinger and J. Michl, Excited states and photochemistry of organic molecules (VCH Publishers, New York, 1995). Thus, the energy transfer of Eq. (1) is not possible via Dexter mechanism. In the second mechanism of Förster transfer (T. Förster, Zwischenmolekulare Energiewanderung and Fluoreszenz, Annalen der Physik, 1948, 2, 55-75; T. Förster, Fluoreszenz organischer Verbindugen (Vandenhoek and Ruprecht, Gottinghen, 1951 ), the energy transfer of Eq. (1) is possible. In Förster transfer, similar to a transmitter and an antenna, dipoles on the donor and acceptor molecules couple and energy may be transferred. Dipoles are generated from allowed transitions in both donor and acceptor molecules. This typically restricts the Förster mechanism to transfers between singlet states.

Nevertheless, as long as the phosphor can emit light due to some perturbation of the state such as due to spin-orbit coupling introduced by a heavy metal atom, it may participate as the donor in Förster transfer. The efficiency of the process is determined by the luminescent efficiency of the phosphor (F Wilkinson, in Advances in Photochemistry (eds. W.A. Noyes, G. Hammond, and J.N. Pitts, pp. 241-268, John Wiley & Sons, New York, 1964), i.e. if a radiative transition is more probable than a non-radiative decay, then energy transfer will be efficient. Such triplet-singlet transfers were predicted by Förster (T. Förster,"Transfer mechanisms of electronic excitation," Discussions of the Faraday Society, 1959,27,7-17) and confirmed by Ermolaev and Sveshnikova (V.L. Ermolaev and E. B. Sveshnikova, "Inductive-resonance transfer of energy from aromatic molecules in the triplet state," Doklady Akademii Nauk SSSR, 1963, 149, 1295-1298), who detected the energy transfer using a range of phosphorescent donors and fluorescent acceptors in rigid media at 77K or 90K. Large transfer distances are observed; for example, with triphenylamine as the donor and chrysoidine as the acceptor, the interaction range is 52Å.

The remaining condition for Förster transfer is that the absorption spectrum should overlap the emission spectrum of the donor assuming the energy levels between the excited and ground state molecular pair are in resonance. In Example 1 of this application, we use the green phosphor fac tris(2-phenylpyridine) iridium (Ir(ppy)₃; M. A. Baldo, et al., Appl. Phys. Lett., 1999, 75, 4-6) and the red fluorescent dye [2-methyl-6-[2-(2,3,6,7-tetrahydro-1H,5H-benzo[ij]quinolizin-9-yl) ethenyl]-4H-pyran-ylidene] propane-dinitrile] ("DCM2"; C. W. Tang, S. A. VanSlyke, and C. H. Chen, "Electroluminescence of doped organic films," J. Appl. Phys., 1989,65,3610-3616). DCM2 absorbs in the green, and, depending on the local polarization field (V. Bulovic, et al., "Bright, saturated, red-to-yellow organic light-emitting devices based on polarization-induced spectral shifts," Chem. Phys. Lett., 1998, 287,455-460), it emits at wavelengths between λ=570 nm and λ=650 nm.

It is possible to implement Förster energy transfer from a triplet state by doping a fluorescent guest into a phosphorescent host material. Unfortunately, such systems are affected by competitive energy transfer mechanisms that degrade the overall efficiency. In particular, the close proximity of the host and guest increase the likelihood of Dexter transfer between the host to the guest triplets. Once excitons reach the guest triplet state, they are effectively lost since these fluorescent dyes typically exhibit extremely inefficient phosphorescence.

To maximize the transfer of host triplets to fluorescent dye singlets, it is desirable to maximize Dexter transfer into the triplet state of the phosphor while also minimizing transfer into the triplet state of the fluorescent dye. Since the Dexter mechanism transfers energy between neighboring molecules, reducing the concentration of the fluorescent dye decreases the probability of triplet-triplet transfer to the dye. On the other hand, long range Förster transfer to the singlet state is unaffected. In contrast, transfer into the triplet state of the phosphor is necessary to harness host triplets, and may be improved by increasing the concentration of the phosphor.

### II.B.2. Interrelation of device structure and emission

Devices whose structure is based upon the use of layers of organic optoelectronic materials generally rely on a common mechanism leading to optical emission. Typically, this mechanism is based upon the radiative recombination of a trapped charge. Specifically, OLEDs are comprised of at least two thin organic layers separating the anode and cathode of the device. The material of one of these layers is specifically chosen based on the material's ability to transport holes, a "hole transporting layer" (HTL), and the material of the other layer is specifically selected according to its ability to transport electrons, an "electron transporting layer" (ETL). With such a construction, the device can be viewed as a diode with a forward bias when the potential applied to the anode is higher than the potential applied to the cathode. Under these bias conditions, the anode injects holes (positive charge carriers) into the hole transporting layer, while the cathode injects electrons into the electron transporting layer. The portion of the luminescent medium adjacent to the anode thus forms a hole injecting and transporting zone while the portion of the luminescent medium adjacent to the cathode forms an electron injecting and transporting zone. The injected holes and electrons each migrate toward the oppositely charged electrode. When an electron and hole localize on the same molecule, a Frenkel exciton is formed. Recombination of this short-lived state may be visualized as an electron dropping from its conduction potential to a valence band, with relaxation occurring, under certain conditions, preferentially via a photoemissive mechanism. Under this view of the mechanism of operation of typical thin-layer organic devices, the electroluminescent layer comprises a luminescence zone receiving mobile charge carriers (electrons and holes) from each electrode.

As noted above, light emission from OLEDs is typically via fluorescence or phosphorescence. There are issues with the use of phosphorescence. It has been noted that phosphorescent efficiency can decrease rapidly at high current densities. It may be that long phosphorescent lifetimes cause saturation of emissive sites, and triplet-triplet annihilation may also produce efficiency leases Another difference between fluorescence and phosphorescence is that energy transfer of triplets from a conductive host to a luminescent guest molecule is typically slower than that of singlets; the long range dipole-dipole coupling (Förster transfer) which dominates energy transfer of singlets is (theoretically) forbidden for triplets by the principle of spin symmetry conservation. Thus, for triplets, energy transfer typically occurs by diffusion of excitons to neighboring molecules (Dexter transfer); significant overlap of donor and acceptor excitonic wavefunctions is critical to energy transfer. Another issue is that triplet diffusion lengths are typically long (e.g., >1400Å) compared with typical singlet diffusion lengths of about 200Å. Thus, if phosphorescent devices are to achieve their potential, device structures need to be optimized for triplet properties. In this invention, we exploit the property of long triplet diffusion lengths to improve external quantum efficiency.

Successful utilization of phosphorescence holds enormous promise for organic electroluminescent devices. For example, an advantage of phosphorescence is that all excitons (formed by the recombination of holes and electrons in an EL), which are (in part) triplet-based in phosphorescent devices, may participate in energy transfer and luminescence in certain electroluminescent materials. In contrast, only a small percentage of excitons in fluorescent devices, which are singlet-based, result in fluorescent luminescence.

An alternative is to use phosphorescence processes to improve the efficiency of fluorescence processes.. Fluorescence is in principle 75% less efficient due the three times higher number of symmetric excited states.

### II.C. Background of materials

### II.C.1. Basic heterostructures

Because one typically has at least one electron transporting layer and at least one hole transporting layer, one has layers of different materials, forming a heterostructure. The materials that produce the electroluminescent emission may be the same materials that function either as the electron transporting layer or as the hole transporting layer. Such devices in which the electron transporting layer or the hole transporting layer also functions as the emissive layer are referred to as having a single heterostructure. Alternatively, the electroluminescent material may be present in a separate emissive layer between the hole transporting layer and the electron transporting layer in what is referred to as a double heterostructure. The separate emissive layer may contain the emissive molecule doped into a host or the emissive layer may consist essentially of the emissive molecule.

That is, in addition to emissive materials that are present as the predominant component in the charge carrier layer, that is, either in the hole transporting layer or in the electron transporting layer, and that function both as the charge carrier material as well as the emissive material, the emissive material may be present in relatively low concentrations as a dopant in the charge carrier layer. Whenever a dopant is present, the predominant material in the charge carrier layer may be referred to as a host compound or as a receiving compound. Materials that are present as host and dopant are selected so as to have a high level of energy transfer from the host to the dopant material. In addition, these materials need to be capable of producing acceptable electrical properties for the OLED. Furthermore, such host and dopant materials are preferably capable of being incorporated into the OLED using materials that can be readily incorporated into the OLED by using convenient fabrication techniques, in particular, by using vacuum-deposition techniques.

### II.C.2. Exciton blocking layer

One can have an exciton blocking layer in OLED devices to substantially block the diffusion of excitons, thus substantially keeping the excitons within the emission layer to enhance device efficiency. The material of blocking layer is characterized by an energy difference ("band gap") between its lowest unoccupied molecular orbital (LUMO) and its highest occupied molecular orbital (HOMO) This band gap substantially prevents the diffusion of excitons through the blocking layer, yet has only a minimal effect on the turn-on voltage of a completed electroluminescent device. The band gap is thus preferably greater than the energy level of excitons produced in an emission layer, such that such excitons are not able to exist in the blocking layer. Specifically, the band gap of the blocking layer is at least as great as the difference in energy between the triplet state and the ground state of the host

For a situation with a blocking layer between a hole-conducting host and the electron transporting layer, one seeks the following characteristics, which are listed in order of relative importance.
1. The difference in energy between the LUMO and HOMO of the blocking layer is greater than the difference in energy between the triplet and ground state singlet of the host material.
2. Triplets in the host material are not quenched by the blocking layer.
3. The ionization potential (IP) of the blocking layer is greater than the ionization potential of the host. (Meaning that holes are held in the host.)
4. The energy level of the LUMO of the blocking layer and the energy level of the LUMO of the host are sufficiently close in energy such that there is less than 50% change in the overall conductivity of the device.
5. The blocking layer is as thin as possible subject to having a thickness of the layer that is sufficient to effectively block the transport of excitons from the emissive layer into the adjacent layer.

That is, to block excitons and holes, the ionization potential of the blocking layer should be greater than that of the HTL, while the electron affinity of the blocking layer should be approximately equal to that of the ETL to allow for facile transport of electrons.
[For a situation in which the emissive ("emitting") molecule is used without a hole transporting host, the above rules for selection of the blocking layer are modified by replacement of the word "host" by "emitting molecule."]

For the complementary situation with a blocking layer between a electron-conducting host and the hole-transporting layer one seeks characteristics (listed in order of importance):
1. The difference in energy between the LUMO and HOMO of the blocking layer is greater than the difference in energy between the triplet and ground state singlet of the host material.
2. Triplets in the host material are not quenched by the blocking layer.
3. The energy of the LUMO of the blocking layer is greater than the energy of the LUMO of the (electron-transporting) host. (Meaning that electrons are held in the host.)
4. The ionization potential of the blocking layer and the ionization potential of the host are such that holes are readily injected from the blocker into the host and there is less than a 50% change in the overall conductivity of the device.
5. The blocking layer is as thin as possible subject to having a thickness of the layer that is sufficient to effectively block the transport of excitons from the emissive layer into the adjacent layer.
[For a situation in which the emissive ("emitting") molecule is used without an electron transporting host, the above rules for selection of the blocking layer are modified by replacement of the word "host" by "emitting molecule."]

### II.D. Color

As to colors, it is desirable for OLEDs to be fabricated using materials that provide electroluminescent emission in a relatively narrow band centered near selected spectral regions, which correspond to one of the three primary colors, red, green and blue so that they may be used as a colored layer in an OLED or SOLED. it is also desirable that such compounds be capable of being readily deposited as a thin layer using vacuum deposition techniques so that they may be readily incorporated into an OLED that is prepared entirely from vacuum-deposited organic materials.

U.S. 08/774,333, filed December 23,1996 (allowed), is directed to OLEDs containing emitting compounds that produce a saturated red emission.

### III. SUMMARY OF THE INVENTION

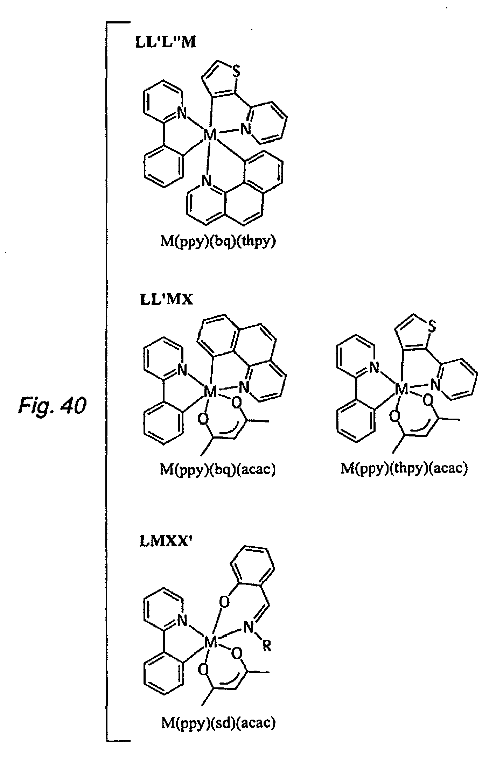

At a general level, the present invention is directed to complexes of a metal M of atomic number greater than 40, wherein M forms an octahedral complex with three bidentate ligands. The metal includes main group metals such as Sb, transition metals of the second row of the transition series of the periodic table, preferably transition metals of the third row of the transition series of the periodic table, and most preferably Ir and Pt. The organometallic complex can be used in the emitter layer of organic light emitting diodes. The complex may be depicted as L L' L" M, wherein L, L', and L" represent the bidentate ligands and M the metal. Examples where all ligands are different are given in Figure 40.

The invention is further directed to organometallic complexes of metal species M with a bidentate monoanionic ligand in which M is coordinated with an sp² hybridized carbon and a heteroatom of the ligand. The complex may be of the form L₃M (wherein each ligand L species is the same), L L' L" M (wherein each ligand species L, L', L"is distinct), or L₂MX, wherein X is a monoanionic bidentate ligand. It is generally expected that the ligand L participates more in the emission process than does X.. Preferably, M is a third row transition metal and most preferably M is Ir or Pt. The invention is directed to meridianal isomers of L₃M wherein the heteroatoms (such as nitrogen) of two ligands L are in a trans configuration. In the embodiment in which M is coordinated with an sp² hybridized carbon and a heteroatom of the ligand, it is preferred that the ring comprising the metal M, the sp² hybridized carbon and the heteroatom contain 5 or 6 atoms.

Furthermore, the present invention is directed to the use of complexes of transition metal species M with bidentate ligands L and M in compounds of formula L₂MX in the emitter layer of organic light emitting diodes. A preferred embodiment is compounds of formula L₂IrX, wherein L and X are distinct bidentate ligands, as dopants in a host layer comprising to function as a emitter layer in organic light emitting diodes.

The present invention is directed to an improved synthesis of organometallic molecules which function as emitters in light emitting devices. The compounds of this invention can be made according to the reaction

L₂M(µ-Cl)₂ML₂ + XH → L₂MX + HCl

wherein
L₂M(µ-Cl)₂ML₂ is a chloride bridged dimer with L a bidentate ligand, and M a metal such as Ir,
XH is a Bronsted acid which reacts with bridging chloride and serves to introduce a bidentate ligand X, where XH can be, for example, acetylacetone, 2-picolinic acid, or N-methylsalicyclanilide. The method involves combining the L₂M(µ-Cl)₂ML₂ chloride bridged dimer with the XH entity.
L₂MX has approximate octahedral disposition of the bidentate ligands L, L, and X about M.

The present invention is further directed to the use of compounds of formula L₂MX as phosphorescent emitters in organic light emitting devices. For example, the compound wherein L=(2-phenylbenzothiazole), X=acetylacetonate, and M=Ir (abbreviated as BTIr) when used as a dopant in CBP (at a level 12% by mass) to form an emitter layer in an OLED shows a quantum efficiency of 12%.
For reference, the formula of 4,4'-N,N'-dicarbazole-biphenyl (CBP) is

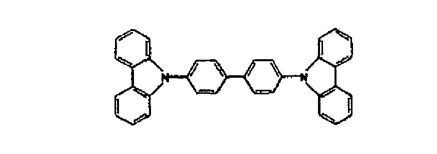

The present invention is further directed to the organometallic complex L₂MX wherein L, by itself, is fluorescent but the resultant L₂MX is phosphorescent. One specific example of this is where L=coumarin-6.

The present invention is further directed to the appropriate selection of L and X to allow color tuning of the complex L₂MX relative to L₃M. For example, Ir(ppy)₃ and (PPy)₂Ir(acac) both give strong green emission with a λₘₐₓ of 510 nm [ppy denotes phenyl pyridine]. However, if the X ligand is formed from picolinic acid instead of from acetylacetone, there is a small blue shift of about 15 nm.

The present invention is further directed to a selection of X such that it has a certain HOMO level relative to the L₃M complex so that carriers (holes or electrons) might be trapped on X (or on L) without a deterioration of emission quality. In this way, carriers (holes or electrons) which might otherwise contribute to deleterious oxidation (or reduction) of the phosphor would be impeded. The carrier that is remotely trapped could readily recombine with the opposite carrier either intramolecularly or with the carrier from an adjacent molecule.

### IV. BRIEF DESCRIPTION OF THE DRAWINGS

Figure 1. Expected structure of L₂IrX complexes along with the structure expected for PPIr. Four examples of X ligands used for these complexes are also shown. The structure shown is for an acac derivative. For the other X type ligands the O-O ligand would be replaced with an N-O ligand.
Figure 2. Comparison of facial and meridianal isomers of L₃M.
Figure 3. Molecular formulae of mer isomers disclosed herewith: mer-Ir(ppy)₃ and mer-Ir(bq)₃. PPY (or ppy) denotes phenyl pyridyl and BQ (or bq) denotes 7,8-benzoquinoline.
Figure 4. Models of mer-Ir(ppy)₃ and (ppy)₂Ir(acae).
Figure 5. (a)Electroluminescent device data (quantum efficiency vs. current density) for 12% by mass "BTIr" in CBP. BTIr stands for bis (2-phenylbenzothiazole) iridium acetylacetonate. (b) emission spectrum from device
Figure 6. Representative molecule to trap holes.
Figure 7. Emission spectrum of Ir(3-MeOppy)₃.
Figure 8. Emission spectrum of tpyIrsd.
Figure 9. Proton NMR spectrum of tpyIrsd (=typIrsd).
Figure 10. Emission spectrum of thpyIrsd.
Figure 11. Proton NMR spectrum of tbpyrIrsd.
Figure 12. Emission spectrum of btIrsd.
Figure 13. Proton NMR spectrum of btIrsd.
Figure 14. Emission spectrum of BQIr.
Figure 15. Proton NMR of BQIr.
Figure 16. Emission spectrum of BQIrFA.
Figure 17. Emission spectrum of THIr (=thpy; THPIr).
Figure 18. Proton NMR spectrum of THPIr.
Figure 19. Emission spectra of PPIr.
Figure 20. Proton NMR spectrum of PPIr.
Figure 21. Emission spectrum of BTHPIr(=BTPIr)
Figure 22. Emission spectrum of tpyIr.
Figure 23. Crystal structure of tpyIr showing trans arrangement of nitrogen.
Figure 24. Emission spectrum of C6,
Figure 25. Emission spectrum of C6Ir.
Figure 26. Emission spectrum of PZIrP.
Figure 27. Emission spectrum of BONIr.
Figure 28. Proton NMR of BONIr.
Figure 29. Emission spectrum of BTIr.
Figure 30. Proton NMR of BTIr.
Figure 31. Emission spectrum of BOIr.
Figure 32. Proton NMR of BOIr.
Figure 33. Emission spectrum of BTIrQ.
Figure 34. Proton NMR spectrum of BTIrQ.
Figure 35. Emission spectrum of BTIrP.
Figure 36. Emission spectrum of BOIrP.
Figure 37. Emission of btIr type complexes with different ligands. Figure 38. Proton NMR of mer-Irbq.
Figure 39. Other suitable L and X ligands for L2MX compounds.
Figure 40. Examples of L L' L" M compounds.

### V. DETAILED DESCRIPTION OF THE INVENTION

### V.A. Chemistry

This invention is directed to the synthesis and use of certain organometallic molecules of formula L₂MX which may be doped into a host phase in an emitter layer of an organic light emitting diode. Optionally, the molecules of formula L₂MX may be used at elevated concentrations or neat in the emitter layer. This invention is further directed to an organic light emitting device comprising an emitter layer comprising a molecule of the formula L₂MX wherein L and X are inequivalent, bidentate ligands and M is a metal, preferably selected from the third row of the transition elements of the periodic table, and most preferably Ir or Pt, which forms octahedral complexes
and wherein the emitter layer produces an emission which has a maximum at a certain wavelength λₘₐₓ.

### V.A.I. Dopants

The general chemical formula for the molecules which are doped into the host phase is L₂MX, wherein M is a transition metal ion which forms octahedral complexes, L is a bidentate ligand, and X is a distinct bidentate ligand.

Examples of L are 2-(1-naphthyl)benzoxazole), (2-phenylbenzoxazole), (2-phenylbenzothiazole), (2-phenylbenzothiazole), (7,8-benzoquinoline), coumarin, (thienylpyridine), phenylpyridine, benzothienylpyridine, 3-methoxy-2-phenylpyridine, thienylpyridine, and tolylpyridine.

Examples of X are acetylacetonate ("acac"), hexafluoroacetylacetonate, salicylidene, picolinate, and 8-hydroxyquinolinate.

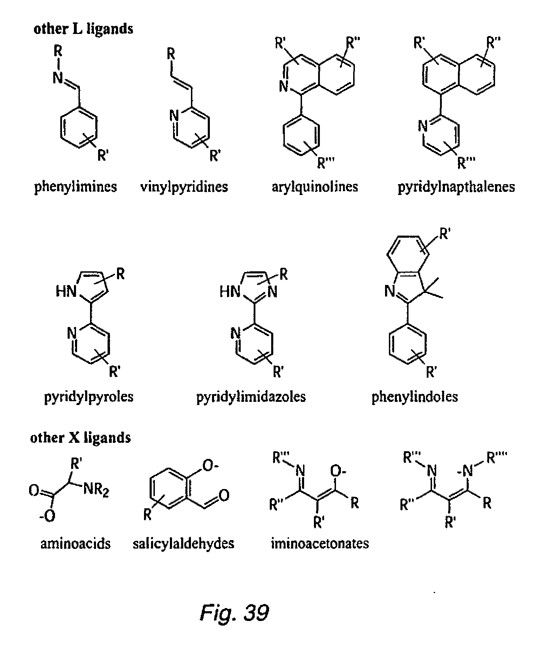

Further examples of L and X are given in Figure 39 and still further examples of L and X may be found in Comprehensive Coordination Chemistry, Volume 2, G. Wilkinson (editor-in-chief), Pergamon Press, especially in chapter 20.1 (beginning at page 715) by M. Calligaris and L. Randaccio and in chapter 20.4 (beginning at page 793) by R. S. Vagg.

### V.A.2. Synthesis of molecules of formula L₂MX

### V.A.2.a. Reaction scheme

The compounds of formula L₂MX can be made according to the reaction

L₂M(µ-C)₂ML₂ + XH → L₂MX + HCl

wherein
L₂M(µ-Cl)₂ML₂ is a chloride bridged dimer with L a bidentate ligand, and M a metal such as Ir,
XH is a Bronsted acid which reacts with bridging chloride and serves to introduce a bidentate ligand X, where XH can be, for example, acetylacetone, hexafluoroacetylacetone, 2-picolinic acid, or N-methylsalicyclanilide.
L₂MX has approximate octahedral disposition of the bidentate ligands L, L, and X about M.

### V.A.2.b. Examples

L₂Ir(µ-Cl)₂IrL₂ complexes were prepared from IrCl₃•nH₂O and the appropriate ligand by literature procedures (S. Sprouse, K. A. King, P. J. Spellane, R. J. Watts, J. Am. Chem. Soc., 1984, 106, 6647-6653; for general reference: G. A. Carlson, et al., Inorg. Chem., 1993, 32, 4483; B. Schmid, et al., Inorg. Chem., 1993, 33, 9; F. Garces, et al.; Inorg. Chem.., 1988,27,3464: M. G. Colombo, et al., Inorg. Chem., 1993, 32, 3088; A. Mamo, et al., Inorg. Chem., 1997, 36, 5947; S. Serroni, et al.; J. Am. Chem. Soc., 1994, 116, 9086; A. P. Wilde, et al., J. Phys. Chem., 1991, 95, 629; J. H. van Diemen, et al., Inorg. Chem., 1992, 31, 3518; M. G, Colombo, et al., Inorg. Chem., 1994, 33, 545)

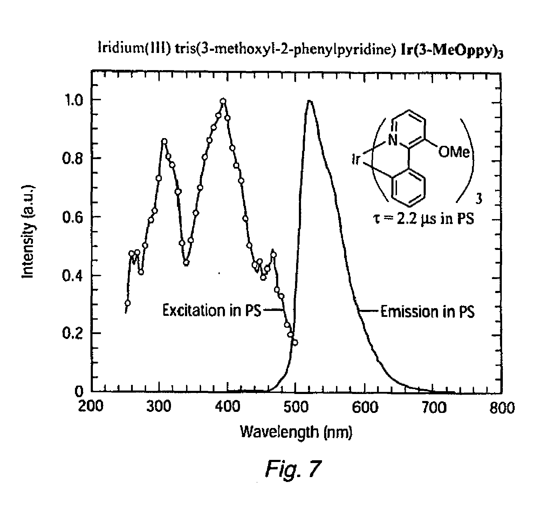

**Ir(3-MeOppy)₃. Ir(atac)₃** (0.57 g, 1.17 mmol) and 3-methoxy-2-phenylpyridine (1.3 g, 7.02 mmol) were mixed in 30 ml of glycerol and heated to 200°C for 24 hrs under N₂. The resulting mixture was added to 100 ml of 1 M HCl. The precipitate was collected by filtration and purified by column chromatography using CH₂Cl₂ as the eluent to yield the product as bright yellow solids (0.35 g, 40%). MS (EI): m/z (relative intensity) 745 (M⁺, 100). 561 (30), 372 (35). Emission spectrum in Figure 7.

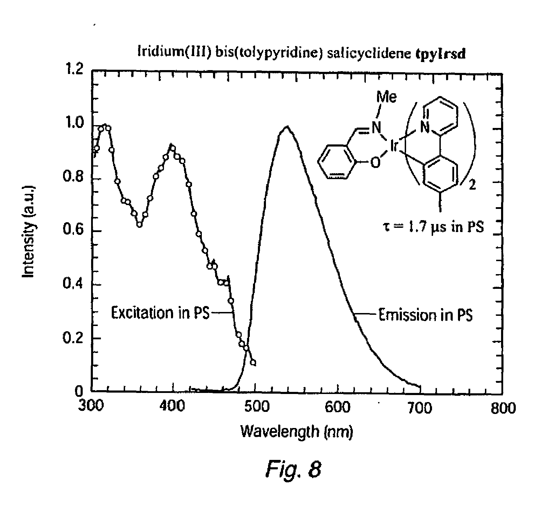

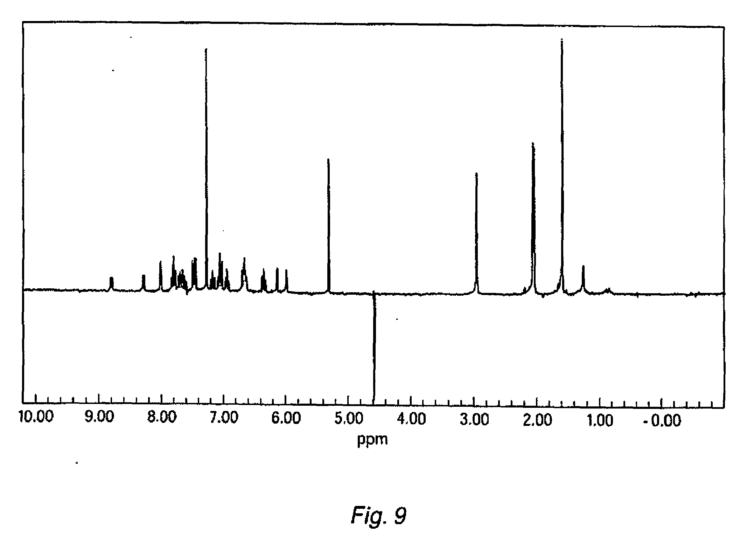

**tpyIrsd.** The chloride bridge dimer (tpyIrCl)₂ (0.07 g, 0.06 mmol), salicylidene (0.022 g, 0.16 mmol) and Na₂CO₃ (0.02 g, 0.09 mmol) were mixed in 10 ml of 1,2-dichloroethane and 2 ml of ethanol. The mixture was refluxed under N₂ for 6 hrs or until no dimer was revealed by TLC. The reaction was then cooled and the solvent evaporated. The excess salicylidene was removed by gentle heating under vacuum. The residual solid was redissolved in CH₂Cl₂ and the insoluble inorganic materials were removed by filtration. The filtrate was concentrated and column chromatographed using CH₂Cl₂ as the eluent to yield the product as bright yellow solids (0.07 g, 85%). MS (EI): m/z (relative intensity) 663 (M⁺, 75), 529 (100), 332 (35). The emission spectrum is in Figure 8 and the proton NMR spectrum is in Figure 9.

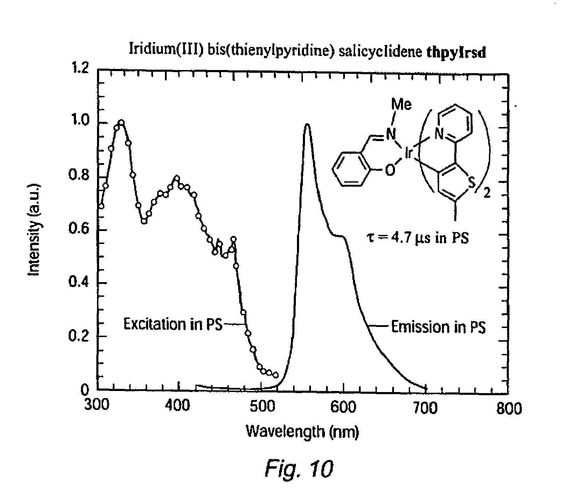

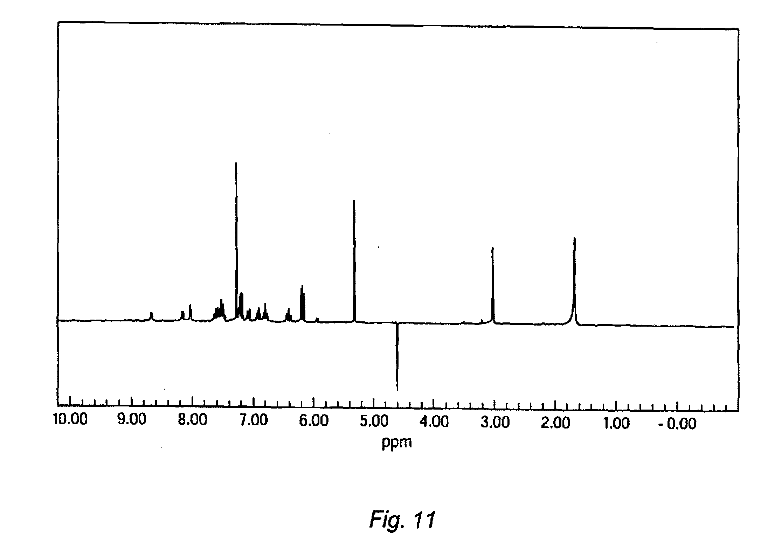

**thpyIrsd.** The chloride bridge dimer (thpyIrCl)₂ (0.21 g, 0.19 mmol) was treated the same way as (tpyIrCl)₂. Yield: 0.21 g, 84%. MS (El): m/z (relative intensity) 647 (M⁺, 100), 513 (30), 486 (15), 434 (20), 324 (25). The emission spectrum is in Figure 10 and the proton NMR spectrum is in Figure 11.

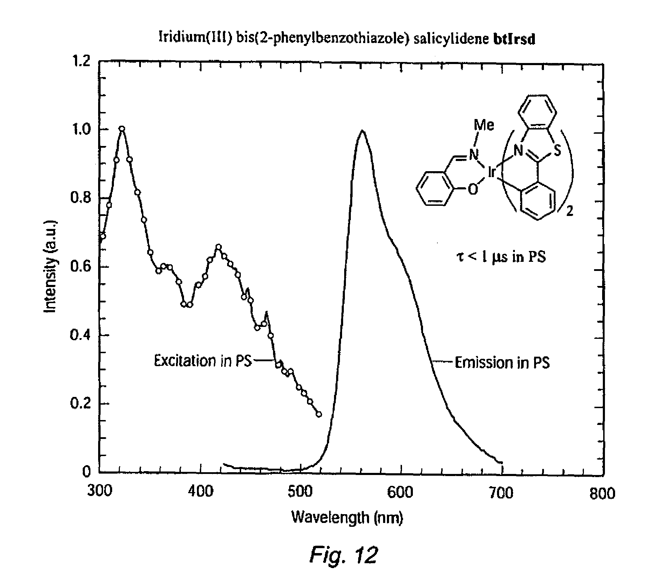

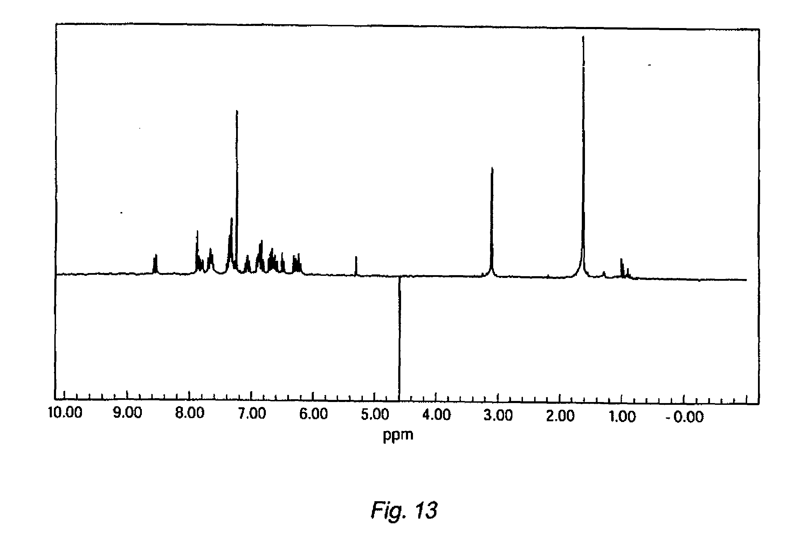

btIrsd. The chloride bridge dimer (btIrCl)₂ (0.05 g, 0.039 mmol) was treated the same way as (tpyIrCl)₂. Yield: 0.05 g, 86%. MS (EI): m/z (relative intensity) 747 (M⁺, 100), 613 (100), 476 (30), 374 (25), 286 (32). The emission spectrum is in Figure 12 and the proton NMR spectrum is in Figure 13.

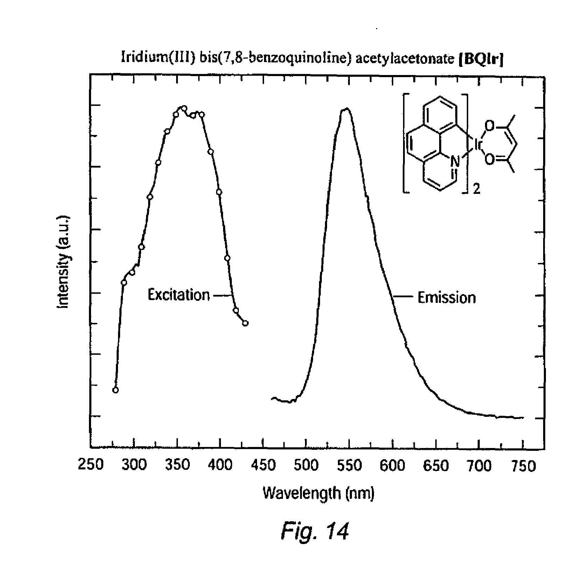

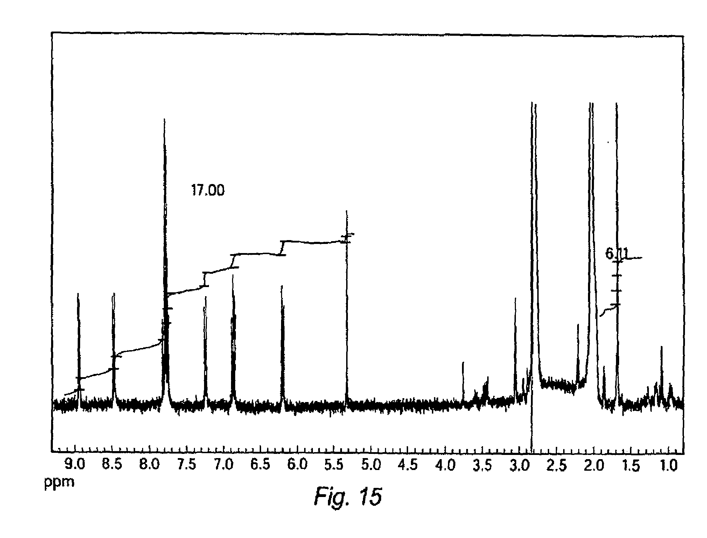

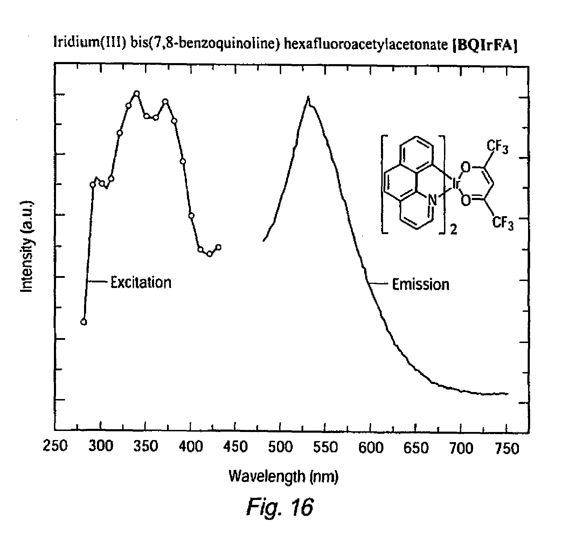

**Ir(bq)₂(acne), BQIr**. The chloride bridged dimer (Ir(bq)₂Cl)₂ (0.091 g, 0.078 mmol), acetylacetone (0.021 g) and sodium carbonate (0.083 g) were mixed in 10 ml of 2-ethoxyethanol. The mixture was refluxed under N₂ for 10 hrs or until no dimer was revealed by TLC. The reaction was then cooled and the yellow precipitate filtered. The product was purified by flash chromatography using dichloromethane. Product: bright yellow solids (yield 91%). ¹H NMR (360 MHz, acetone-d₆), ppm: 8.93 (d,2H), 8.47 (d,2H), 7.78 (m,4H), 7.25 (d,2H), 7.15 (d,2H), 6.87 (d,2H), 6.21 (d,2H), 5.70 (s, 1H), 1.63 (s,6H). MS, e/z: 648 (M+,80%), 549 (100%). The emission spectrum is in Figure 14 and the proton NMR spectrum is in Figure 15. **Ir(bq)₂(Facac),BQIrFA**. The chloride bridged dimer (Ir(bq)₂Cl)₂ (0.091g, 0.078 mmol), hexafluoroacetylacetone (0.025 g) and sodium carbonate (0.083 g) were mixed in 10 ml of 2-ethoxyethanol. The mixture was refluxed under N₂ for 10 hrs or until no dimer was revealed by TLC. The reaction was then cooled and the yellow precipitate filtered. The product was purified by flash chromatography using dichloromethane. Product: yellow solids (yield 69%). ¹H NMR (360 MHz, acetone-d₆), ppm: 8.99 (d,2H), 8.55 (d,2H), 7.86 (m,4H), 7.30 (d,2H), 7.14 (d,2H), 6.97 (d,2H), 6.13 (d,2H), 5.75 (s,1H). MS, e/z: 684 (M+,59%), 549 (100%). Emission spectrum in Figure 16.

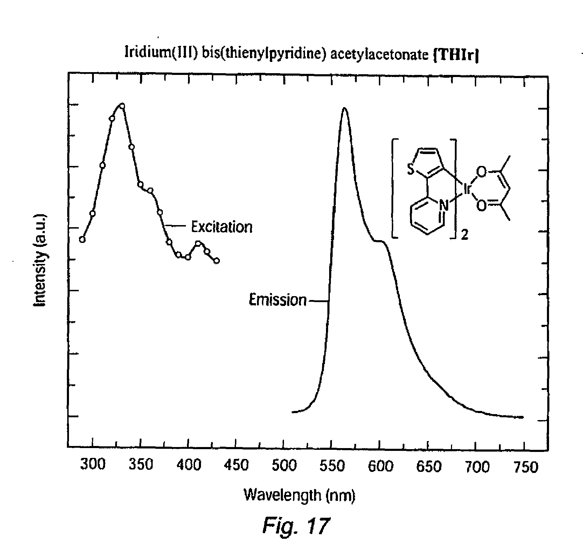

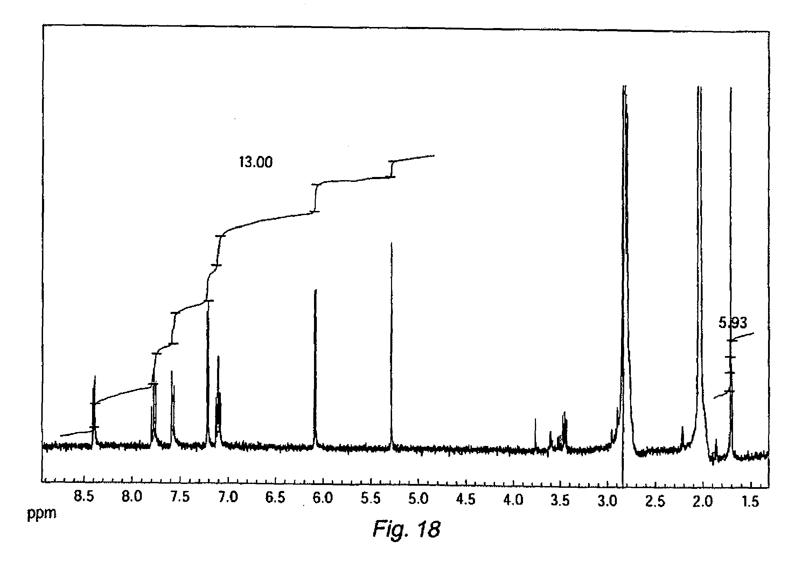

**Ir(thpy)₂(acae), THPIr**. The chloride bridged dimer (Ir(thpy)₂Cl)₂ (0.082 g, 0.078 mmol), acetylacetone (0.025 g) and sodium carbonate (0.083 g) were mixed in 10 ml of 2-ethoxyethanol. The mixture was refluxed under N₂ for 10 hrs or until no dimer was revealed by TLC. The reaction was then cooled and the yellow precipitate filtered. The product was purified by flash chromatography using dichloromethane. Product: yellow-orange solid (yield 80%). ¹H NMR (360 MHz, acetone-d₆), ppm: 8.34 (d,2H), 7.79 (m,2H), 7.58 (d,2H), 7.21 (d,2H), 7.15 (d,2H), 6.07 (d,2H), 5.28 (s.1H. 1.70 (s,6H). MS, e/z: 612 (M+,89%), 513 (100%). The emission spectrum is in Figure 17 (noted "THIr") and the proton NMR spectrum is in Figure 18.

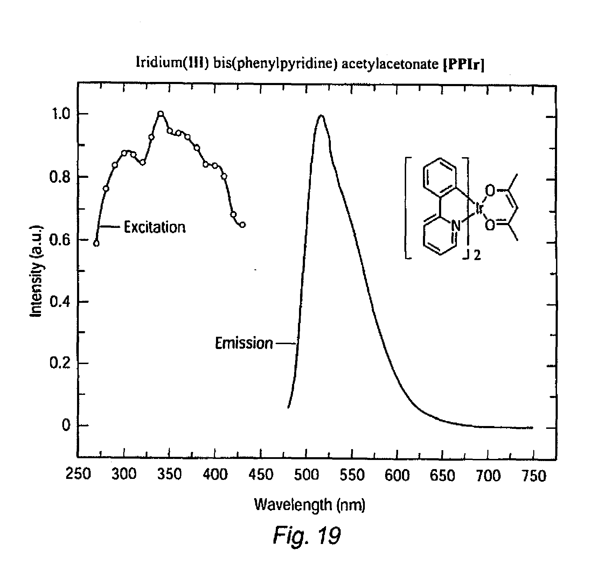

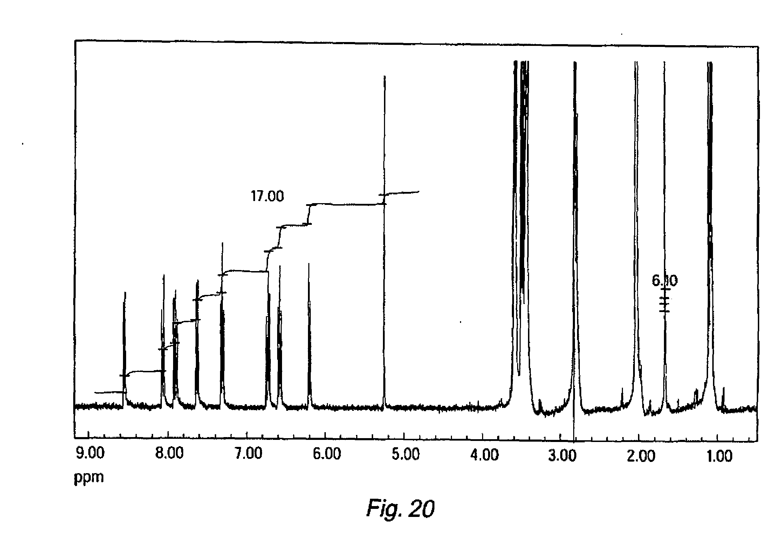

**Ir(ppy)=(acac)**, **PPIr.** The chloride bridged dimer (Ir(ppy)₂Cl)₂ (0.080 g, 0.078 mmol), acetylacetone (0.025 g) and sodium carbonate (0.083 g) were mixed in 10 ml of 2-ethoxyethanol. The mixture was refluxed under N₂ for 10 hrs or until no dimer was revealed by TLC. The reaction was then cooled and the yellow precipitate filtered. The product was purified by flash chromatography using dichloromethane. Product: yellow solid (yield 87%). ¹H NMR (360 MHz, acetone-d₆), ppm: 8.54 (d,2H), 8.06 (d,2H), 7.92 (m,2H), 7.81 (d,2H), 7.35 (d,2H), 6.78 (m,2H), 6.69 (m,2H), 6.20 (d,2H), 5.12 (s,1H, 1.62 (s,6H). MS. e/z: 600 (M+,75%), 501 (100%). The emission spectrum is in Figure 19 and the proton NMR spectrum is in Figure 20.

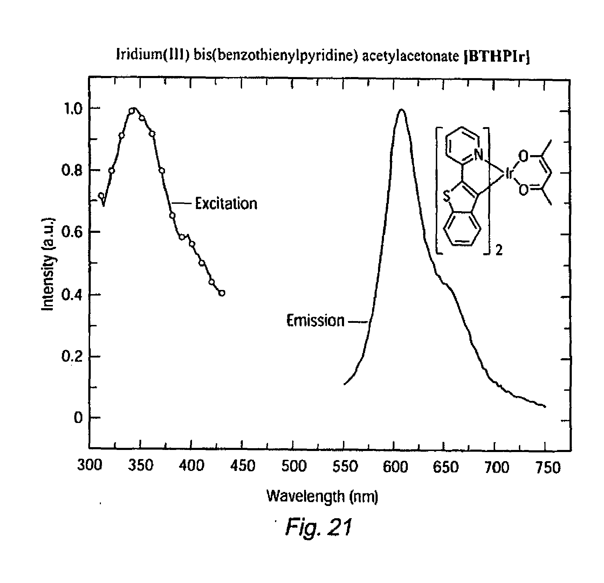

**Ir(bthpy)₂(acac), BTPIr.** The chloride bridged dimer (Ir(bthpy)₂Cl)₂ (0.103 g, 0.078 mmol), acetylacetone (0.025 g) and sodium carbonate (0.083 g) were mixed in 10 ml of 2-ethoxyethanol. The mixture was refluxed under N₂ for 10 hrs or until no dimer was revealed by TLC. The reaction was then cooled and the yellow precipitate filtered. The product was purified by flash chromatography using dichloromethane. Product: yellow solid (yield 49%). MS, e/z: 712 (M+,66%), 613 (100%). Emission spectrum is in Figure 21.

**[Ir(ptpy)₂Cl]₂:** A solution of IrCl₃•xH₂O (1.506g, 5.030 mmol) and 2-(p-tolyl)pyridine (3.509 g, 20.74 mmol) in 2-ethoxyethanol (30 mL) was refluxed for 25 hours. The yellow-green mixture was cooled to room temperature and 20 mL of 1.0 M HCl was added to precipitate the product. The mixture was filtered and washed with 100 mL of 1.0 M HCl followed by 50 mL of methanol then dried. The product was obtained as a yellow powder (1.850 g, 65%).

**[Ir(ppz)₂Cl]₂:** A solution of IrCl₃•xH₂O (0.904 g , 3.027 mmol) and 1-phenylpyrazole (1.725 g, 11.96 mmol) in 2-ethoxyethanol (30 mL) was refluxed for 21 hours. The gray-green mixture was cooled to room temperature and 20 mL of 1.0 M HCl was added to precipitate the product. The mixture was filtered and washed with 100 mL of 1.0 M HCl followed by 50 mL of methanol then dried. The product was obtained as a light gray powder (1.133 g, 73%).

**[Ir(C6)₂Cl]₂:** A solution of IrCl₃•xH₂O (0.075 g, 0.251 mmol) and coumarin C6 [3-(2-benzothiazolyl)-7-(diethyl)coumarin] (Aldrich) (0.350 g, 1.00 mmol) in 2-ethoxyethanol (15 mL) was refluxed for 22 hours. The dark red mixture was cooled to room temperature and 20 mL of 1.0 M HCl was added to precipitate the product. The mixture was filtered and washed with 100 mL of 1.0 M HCl followed by 50 mL of methanol. The product was dissolved in and precipitated with methanol. The solid was filtered and washed with methanol until no green emission was observed in the filtrate. The product was obtained as an orange powder (0.0657 g, 28%).

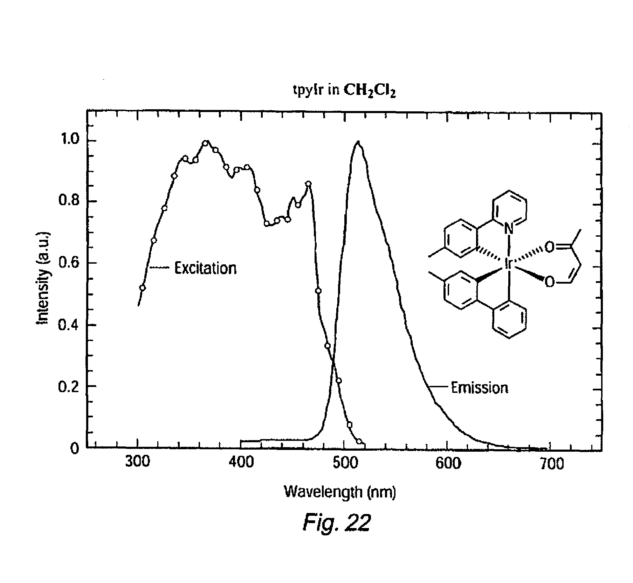

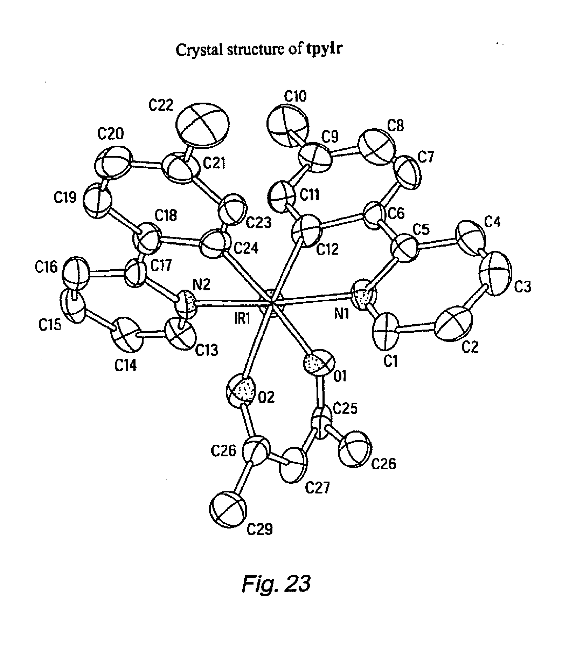

**Ir(ptpy)₂acac (tpyIr):** A solution of [Ir(ptpy)₂Cl]₂ (1.705 g 1.511 mmol), 2,4-pentanedione (3.013g, 30.08 mmol) and (1.802 g" 17-04 mmol) in 1,2-dichloroethane (60 mL) was refluxed for 40 hours. The yellow-green mixture was cooled to room temperature and the solvent was removed under reduced pressure. The product was taken up in 50 mL of CH₂Cl₂ and filtered through Celite. The solvent was removed under reduced pressure to yield orange crystals of the product (1.696 g, 89%). The emission spectrum is given in Figure 22. The results of an x-ray diffraction study of the structure are given in Figure 23. One sees that the nitrogen atoms of the tpy ("tolyl pyridyl") groups are in a trans configuration. For the x-ray study, the number of reflections was 4663 and the R factor was 5.4%.

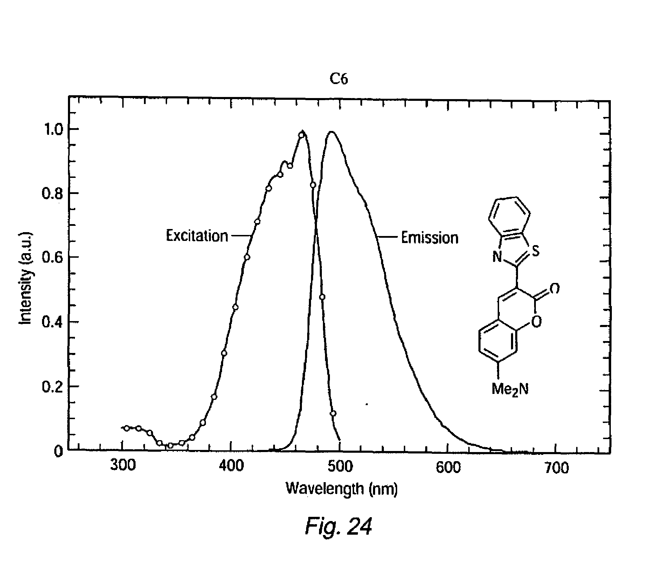

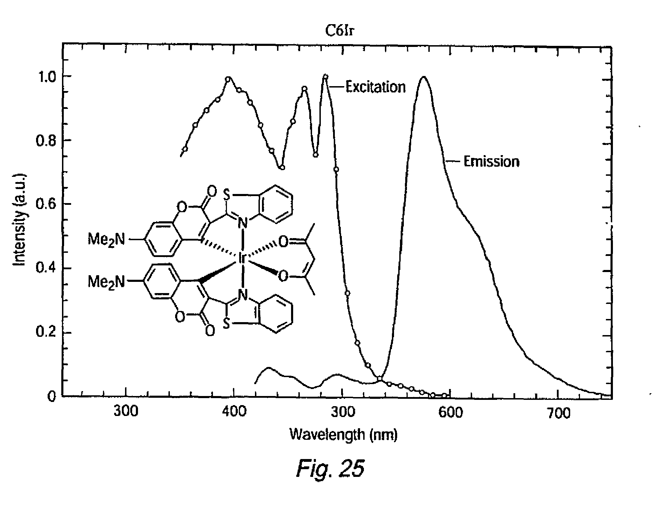

**Ir(C6)₂acac (C6Ir):** Two drops of 2,4-pentanedione and an excess of Na₂CO₃ was added to solution of [Ir(C₆)₂Cl]₂ in CDCl₃. The tube was heated for 48 hours at 50 °C and then filtered through a short plug of Celite in a Pasteur pipet. The solvent and excess 2,4-pentanedione were removed under reduced pressure to yield the product as an orange solid. Emission of C6 in Figure 24 and of C6Ir in Figure 25.

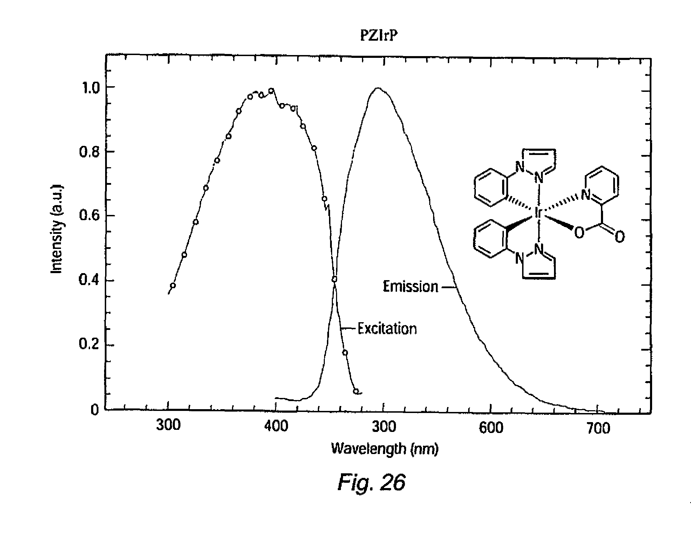

**Ir(ppz)₂plcolinate (PZIrp):** A solution of [Ir(ppz)₂Cl]₂ (0.0545 g, 0.0530 mmol) and picolinic acid (0.0525 g, 0.426 mmol) in CH₂Cl₂ (15 mL) was refluxed for 16 hours. The light green mixture was cooled to room temperature and the solvent was removed under reduced pressure. The resultant solid was taken up in 10 mL of methanol and a light green solid precipitated from the solution. The supernatant liquid was decanted off and the solid was dissolved in CH₂Cl₂ and filtered through a short plug of silica. The solvent was removed under reduced pressure to yield light green crystals of the product (0.0075 g, 12%). Emission in Figure 26.

**2-(1-naphthyl)benzoxazole, (BZO-Naph).** (11.06g. 101 mmol) of2-aminophenol was mixed with (15.867g, 92.2mmol) of 1-naphthoic acid in the presence of polyphosphoric acid. The mixture was heated and stirred at 240°C under N₂ for 8 hrs. The mixture was allowed to cool to 100°C, this was followed by addition of water. The insoluble residue was collected by filtration, washed with water then reslurried in an excess of 10 % Na2CO3. The alkaline slurry was filtered and the product washed thoroughly with water and dried under vacuum. The product was purified by vacuum distillation. BP 140°C /0.3 mmHg. Yield 4.8 g (21%).

**Tetrakis(2-(1-naphthyl)benzoxazole*C*², *N*)(**µ**-dichloro)diiridium. ((Ir₂(BZO-Naph)₄Cl)₂).** Iridium trichloride hydrate (0.388 g) was combined with 2-(1-naphthyl)benzoxazole (1.2 g, 4.88 mmol), The mixture was dissolved in 2-ethoxyethanot (30 mL) then refluxed for 24 hrs. The solution was cooled to room temperature, the resulting orange solid product was collected in a centrifuge tube. The dimer was washed with methanol followed by chloroform through four cycles of centrifuge/redispersion cycles. Yield 0.66g

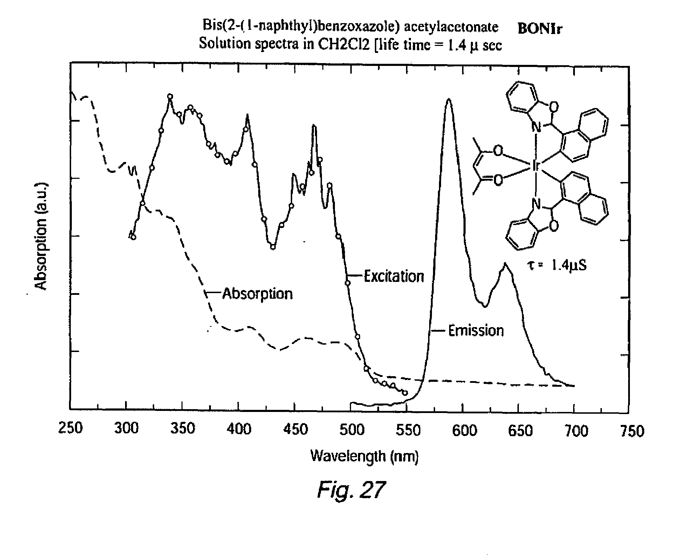

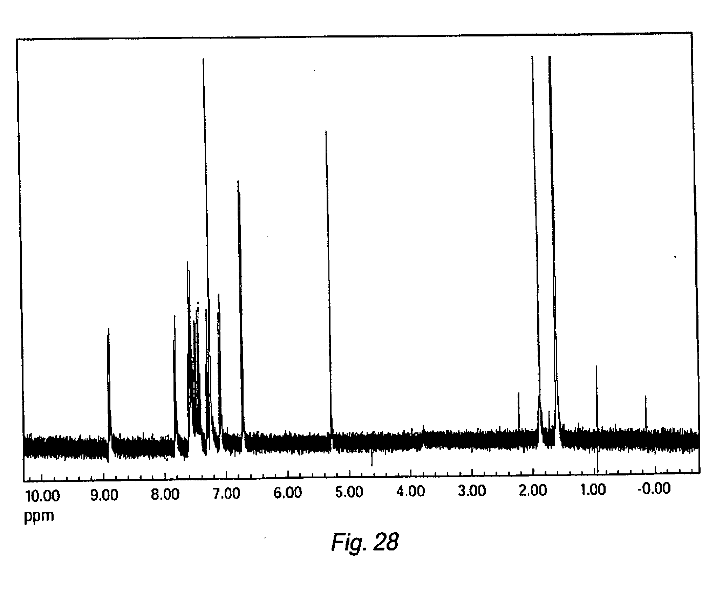

**Bis(2-(1-napbthyl)benzoxazole) acetylacetonate, Ir(BZO-Naph)₂(acac), (BONIr),** The chloride bridged dimer (Ir₂(BZO-Naph)₄Cl)₂ (0.66 g, 0.46 mmol), acetylacetone (0.185 g) and sodium carbonate (0.2 g) were mixed in 20 ml of dichloroethane. The mixture was refluxed under N₂ for 60 hrs. The reaction was then cooled and the orange/red precipitate was collected in centrifuge tube. The product was washed with water/methanol (1:1) mixture followed by methanol wash through four cycles of centrifuge/redispersioncycles. The orange/red solid product was puri fied by sublimation. SP 250°C/2x10⁻³ torr.yield 0.57g (80%). The emission spectrum is in Figure 27 and the proton NMR spectrum is in Figure 28.

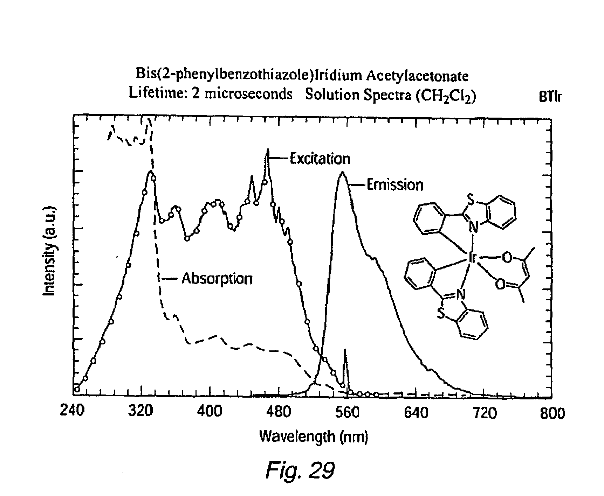

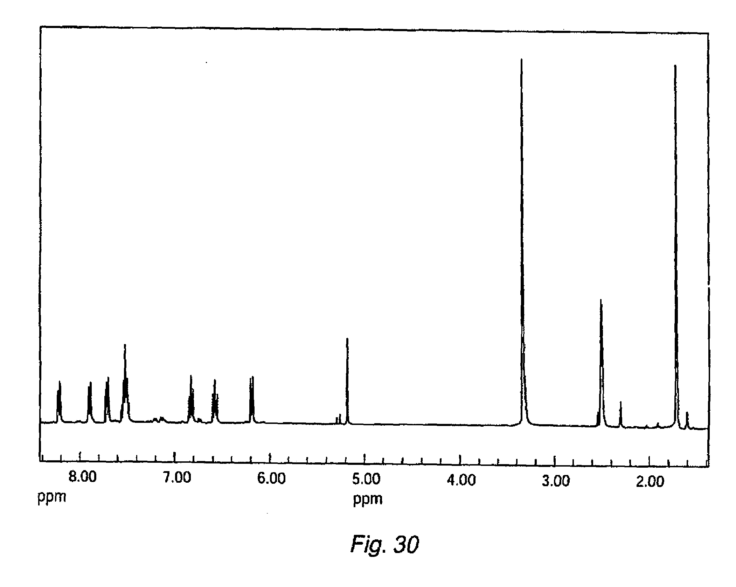

**Bis(2-pbenylbenzothiazole) Iridium acetylacetonate (BTIr);** 9.8 mmol (0.98g, 1.0mL) of 2,4-pentarnedione was added to a room-temperature solution of 2.1 mmol 2-phenylbenzothiazole Iridium chloride dimer (2.7g) in 120mL of 2-ethoxyethanol Approximately 1g of sodium carbonate was added, and the mixture was heated to reflux under nitrogen in an oil bath for several hours. Reaction mixture was cooled to room temperature, and the orange precipitate was filtered off via vacuum. The filtrate was concentrated and methanol was added to precipitate more product. Successive filtrations and precipitations afforded a 75%yield. The emission spectrum is in Figure 29 and the proton NMR spectrum is in Figure 30.

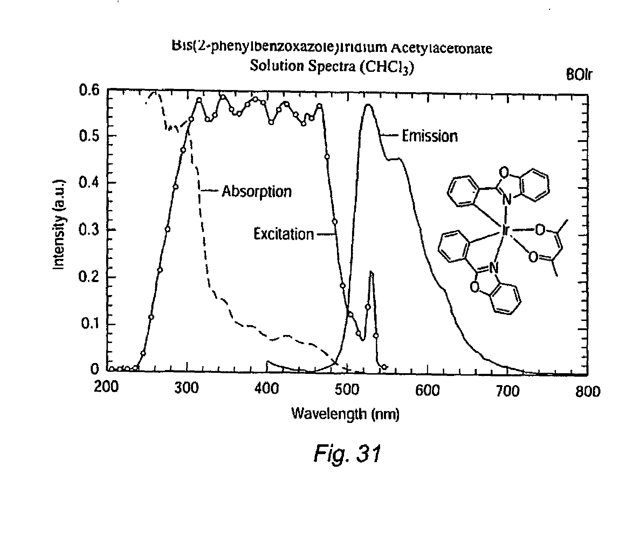

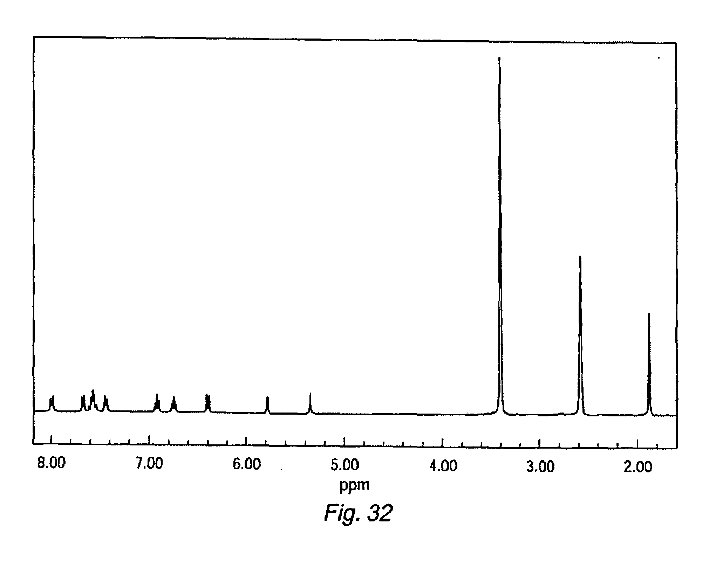

**Bis(2-phenylbenzooxazole) Iridium acac (BOIr):** 9.8 mmol (0.98g, 1.0mL) of 2,4-pentanedione was added to a room-temperature solution of 2.4 mmol 2-phenylbenzoxazole Iridium chloride dimer (3.0g) in 120mL of 2-ethoxyethanol. Approximately 1g of sodium carbonate was added, and the mixture was heated to reflux under nitrogen in an oil bath overnight (∼16hrs.). Reaction mixture was cooled to room temperature, and the yellow precipitate was filtered off via vacuum. The filtrate was concentrated and methanol was added to precipitate more product. Successive filtrations and precipitations afforded a 60%yield. The emission spectrum is in Figure 31and the proton NMR spectrum is in Figure 32.

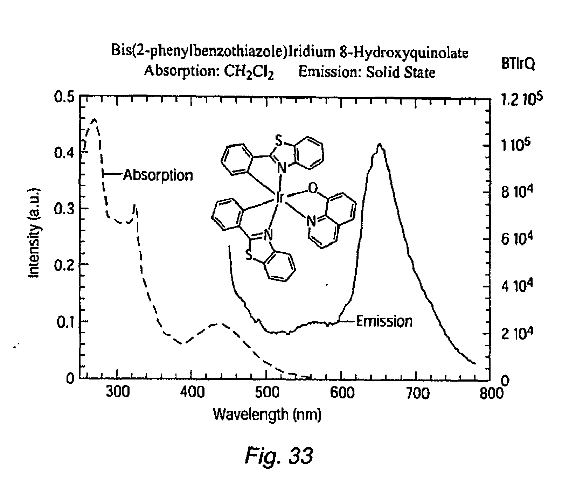

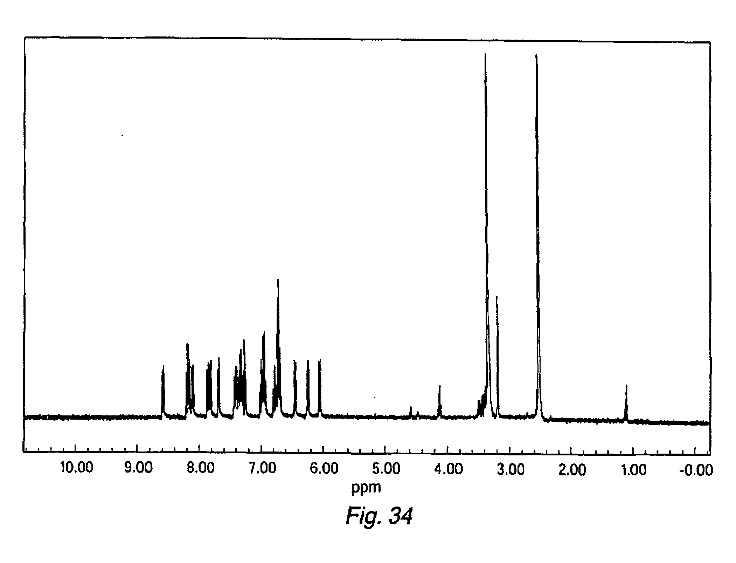

**Bis(2-phenylbenzothiazole) Iridium (8-hydroxyquinolate) (BTIrQ):** 4.7 mmol (0.68g) of 8-hydroxyquinoline was added to a room-temperature solution of 0.14 mmol 2-phenylbenzothiazole Iridium chloride dimer (0.19g) in 20mL of 2-ethoxyethanol. Approximately 700mg of sodium carbonate was added, and the mixture was heated to reflux under nitrogen in an oil bath overnight (23hrs.). Reaction mixture was cooled to room temperature, and the red precipitate was filtered off via vacuum. The filtrate was concentrated and methanol was added to precipitate more product. Successive filtrations and precipitations afforded a 57%yield. The emission spectrum is in Figure 33 and the proton NMR spectrum is in Figure 34.

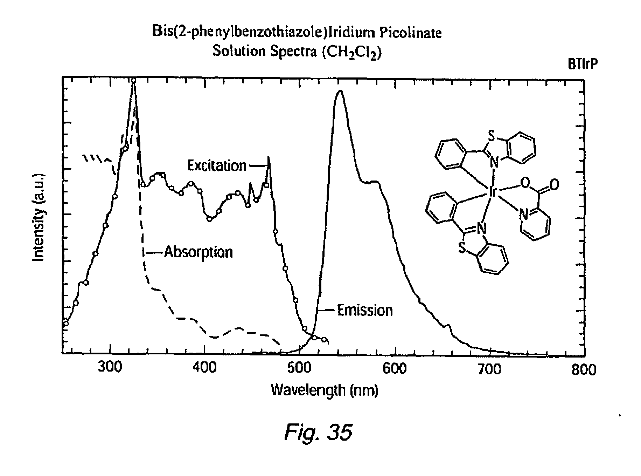

**Bis(2-phenylbenzothiazole) Iridium picolinate (BTIrP):** 2.14 mmol (0.26g) of picolinic acid was added to a room-temperature solution of 0.80 mmol 2-phenylbenzothiazole Iridium chloride dimer (1.0g) in 60mL of dichloromethane. The mixture was heated to reflux under nitrogen in an oil bath for 8.5 hours. The reaction mixture was cooled to room temperature, and the yellow precipitate was filtered off via vacuum. The filtrate was concentrated and methanol was added to precipitate more product. Successive filtrations and precipitations yielded about 900mg of impure product. Emission spectrum is in Figure 35.

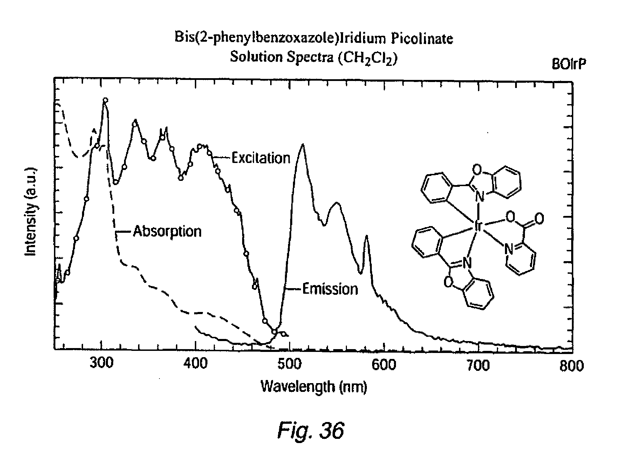

**Bis(2-phenylbenzooxazole) Iridium picolinate (BOIrP):** 0.52 mmol (0.064g) of picolinic acid was added to a room-temperature solution of 0.14 mmol 2-phenylbenzoxazole Iridium chloride dimer (0.18g) in 20mL of dichloromethane. The mixture was heated to reflux under nitrogen in an oil bath overnight (17.5hrs.). Reaction mixture was cooled to room temperature, and the yellow precipitate was filtered off via vacuum. The precipitate was dissolved in dichloromethane and transferred to a vial, and the solvent was removed. Emission spectrum is in Figure 36.

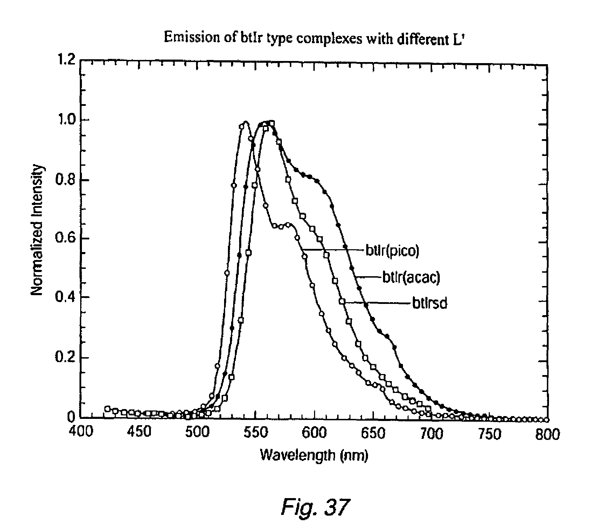

Comparative emission spectra for different L' in btIr complexes are in Figure 37.

### V.A.2.c. Advantages over the prior art

This synthesis has certain advantages over the prior art. Compounds of formula PtL₃ cannot be sublimed without decomposition. Obtaining compounds of formula IrL₃ can be problematic. Some ligands react cleanly with Ir(acac)3 to give the tris complex, but more than half of the ligands we have studied do not react cleanly in the reaction:

3 L + Ir(arac)3 → L₃Ir + acacH

typically 30% yield, L=2-phenylpyridine, benzoquinoline, 2-thienylpyridine A preferred route to Ir complexes can be through the chloride-bridged dimer L₂M(µ-Cl)₂ML₂ via

4 L + IrC13.nH20 → L₂M(µ-Cl)₂ML₂ + 4 Hcl

Although fewer than 10% of the ligands we have studied failed to give the Ir dimer cleanly and in high yield, the conversion of the dimer into the tris complex IrL₃ is problematic working for only a few ligands.

L₂M(µ-Cl)₂ML₂ + 2Ag + 2L → L₃Ir + 2AgCl

We have discovered that a far more fruitful approach to preparing phosphorescent complexes is to use chloride bridged dimers to create emitters. The dimer itself does not emit strongly, presumably because of strong self quenching by the adjacent metal (e.g., iridium) atoms. We have found that the chloride ligands can be replaced by a chelating ligand to give a stable, octahedral metal complex through the chemistry:

L₂M(µ-Cl)₂ML₂ + XH → L₂MX + HCl

We have extensively studied the system wherein M = iridium. The resultant iridium complexes emit strongly, in most cases with lifetimes of 1-3 microseconds ("µsec"). Such a lifetime is indicative of phosphorescence (see Charles Kittel. Introduction to Solid State Physics). The transition in these materials is a metal ligand charge transfer ("MLCT").

In the Detailed Discussion below, we analyze data of emission spectra and lifetimes of a number of different complexes, all of which can be characterized as L₂MX (M=Ir), where L is a cyclometallated (bidentate) ligand and X is a bidentate ligand. In nearly every case, the emission in these complexes is based on an MLCT transition between Ir and the L ligand or a mixture of that transition and an intraligand transition. Specific examples are described below. Based on theoretical and spectroscopic studies, the complexes have an octahedral coordination about the metal (for example, for the nitrogen heterocycles of the L ligand, there is a trans disposition in the Ir octahedron).

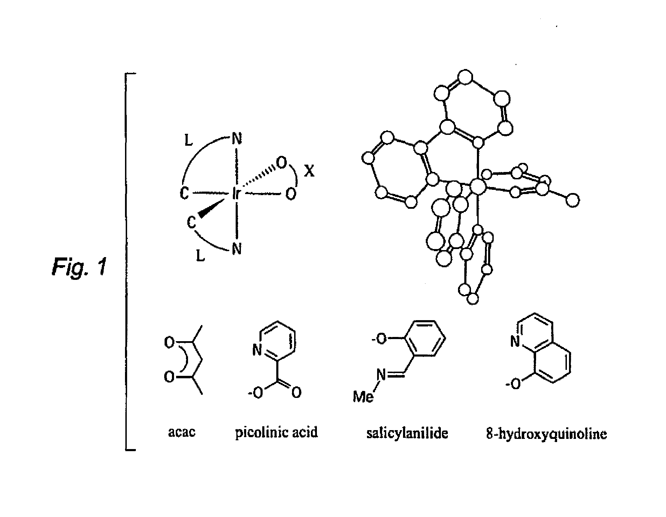

Specifically, in Figure 1, we give the structure for L₂IrX, wherein L = 2-phenyl pyridine and X = acac, picolinate (from picolinic acid), salicylanilide, or 8-hydroxyquinolinate.

### V.A.2.d. Facial vs. meridianal isomers

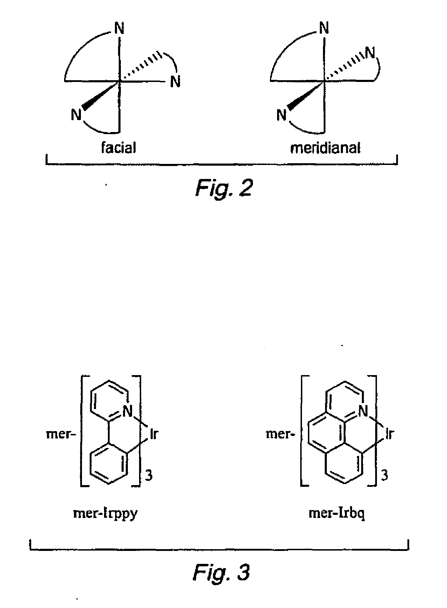

A slight variation of the synthetic route to make L₂IrX allows formation of meridianal isomers of formula L₃Ir. The L₃Ir complexes that have been disclosed previously all have a facial disposition of the chelating ligands. Herewith, we disclose the formation and use of meridianal L₃Ir complexes as phosphors in OLEDs. The two structures are shown in the Figure 2.

The facial L₃Ir isomers have been prepared by the reaction of L with Ir(acac)₃ in refluxing glycerol as described in equation 1 (below). A preferred route into L₃Ir complexes is through the chloride bridged dimer (L₂Ir(µ-Cl)₂IrL₂), equation 2+3 (below). The product of equation 3 is a facial isomer, identical to the one formed from Ir(acac)₃. The benefit of the latter prep is a better yield of facial-L₃Ir. If the third ligand is added to the dimer in the presence of base and acetylacetone (no Ag⁺) a good yield of the meridianal isomer is obtained. The meridianal isomer does not convert to the facial one on recrystallization, refluxing in coordinating solvents or on sublimation. Two examples of these meridianal complexes have been formed, mer-Irppy and mer-Irbq (Figure 3), however, we believe that any ligand that gives a stable facial-L₃Ir can be made into a meridianal form as well.
(1) 3 L + Ir(acac)₃ → facial- L₃Ir + acacH
   typically 30% yield, L = 2-phenylpyridine, bezoquinoline, 2-thienylpyridine
(2) 4 L + IrCl₃•nH₂O → L₂Ir(µ-Cl)₂IrL₂ + 4 HCl
   typically > 90% yield, see attached spectra for examples of L, also works well for all ligands that work in (1)
(3) L₂Ir(µ-Cl)₂IrL₂ + 2 Ag* + 2 L → 2 facial-L₃Ir + 2 AgCl
   typically 30% yield, only works well for the same ligands that work well for (1)
(4) L₂Ir(µ-Cl)₂IrL₂ + XH + Na₂CO₃ + L → merdianal-L₃Ir
   typically > 80% yield, XH = acetylacetone

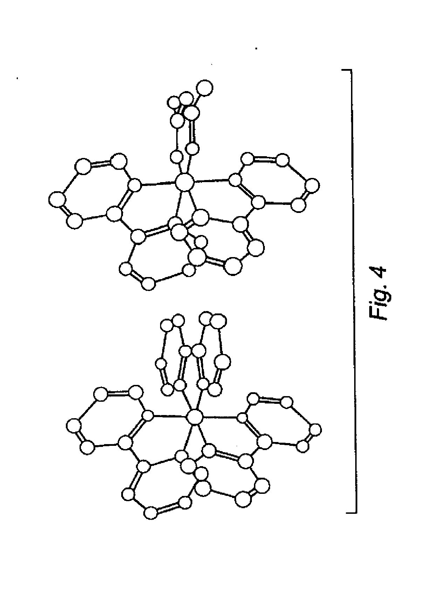

Surprisingly, the photophysics of the meridianal isomers is different from that of the facial forms. This can be seen in the details of the spectra discussed below, which show a marked red shift and broadening in the meridianal isomer relative to its facial counterpart The emission lines appear as if a red band has been added to the and characteristic of the facial-L₃Ir. The structure of the meridianal isomer is similar to those of L₂IrX complexes, with respect, for example, to the arrangement of the N atoms of the ligands about Ir. Specifically, for L=ppy ligands, the nitrogen of the L ligand is trans in both mer-Ir(ppy)₃ and in (ppy)₂Ir(acac). Further, one of the L ligands for the mer-L₃Ir complexes has the same coordination as the X ligand of L₂IrX complexes. In order to illustrate this point a model of mer-Ir(ppy)₃ is shown next to (ppy)₂Ir(acac) in Figure 4. One of the ppy ligands of mer-Ir(ppy)₃ is coordinated to the Ir center in the same geometry as the acac ligand of (ppy)₂Ir(acac).

The HOMO and LUMO energies of these L₃Ir molecules are clearly affected by the choice of isomer. These energies are very important is controlling the current-voltage characteristics and lifetimes of OLEDs prepared with these phosphors.

The syntheses for the two isomers depicted in Figure 3 are as follows.

### Syntheses of meridianal isomers:

***mer*-Irbq:** 91 mg (0.078 mmol) of [Ir(bq)2CL]2 dimer, 35.8 mg (0.2 mmol) of7,8-benzoquinoline, 0.02 ml of acetylacetone (ca. 0.2 mmol) and 83 mg (0.78 mmol) of sodium carbonate were boiled in 12 ml of 2-ethoxyethanol (used as received) for 14 hours in inert atmosphere. Upon cooling yellow-orange precipitate forms and is isolated by filtration and flash chromatography (silica gel, CH2Cl2) (yield 72%). 1H

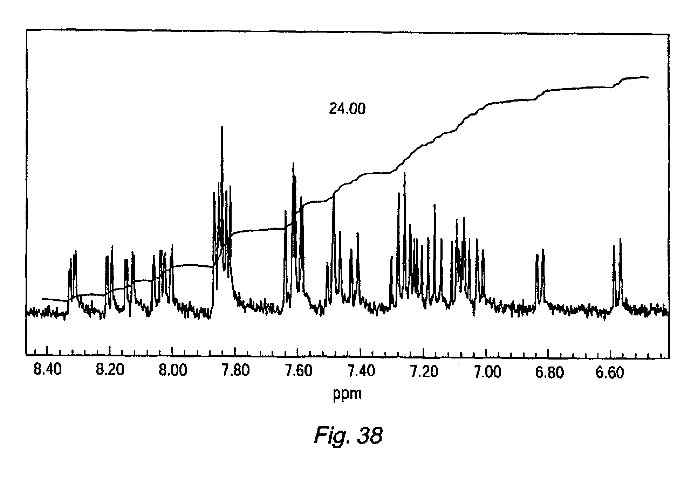

NMR (360 MHz, dichloromethane-d2),ppm: 8.31 (q,1H), 8.18(q,1H), 8.12 (q,1H), 8.03(m,2H), 7.82 (m, 3H), 7.59 (m,2H), 7.47 (m,2H), 7.40 (d,1H), 7.17 (m,9H), 6.81 (d,1H), 6.57 (d,1H). MS,e/z: 727 (100%,M+). NMR spectrum in Figure 38.

**mer-Ir(tpy)₃:** A solution of IrCl₃•xH₂O (0.301 g , 1.0 mmol), 2-(p-tolyl)pyridine (1.027 g, 6.069 mmol), 2,4-pentanedione (0.208 g, 2.08 mmol) and Na₂CO₃ (0.350 g,, 3.30 mmol) in 2-ethoxyethanol (30 mL) was refluxed for 65 hours. The yellow-green mixture was cooled to room temperature and 20 mL of 1.0 M HCl was added to precipitate the product. The mixture was filtered and washed with 100 mL of 1.0 M HCl followed by 50 mL of methanol then dried and the solid was dissolved in CH₂Cl₂ and filtered through a short plug of silica. The solvent was removed under reduced pressure to yield the product as a yellow-orange powder (0.265 g, 38%).

### V.A.3. Potential host molecules

This invention is directed toward the use of the above-noted dopants in a host phase. This host phase may be comprised of molecules comprising a carbazole moiety. Molecules which fall within the scope of the invention are included in the following. 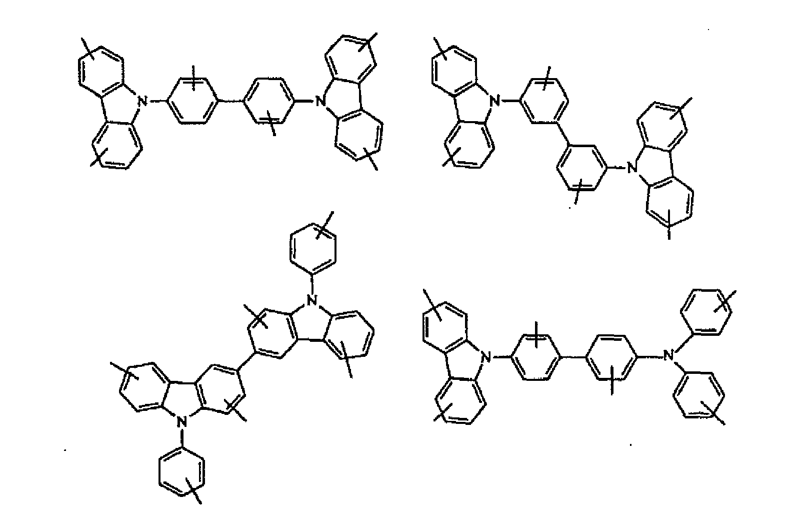 [A line segment denotes possible substitution at any available carbon atom or atoms of the indicated by ring by alkyl or aryl groups.]

An additional preferred molecule with a carbazole functionality is 4,4'-N,N'-dicarbazole-biphenyl (CBP), which has the formula

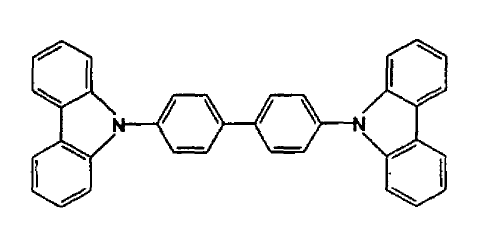

### V.B.1. Utilization in devices

The device structure that we chose to use is very similar to the standard vacuum deposited one. As an overview, a hole transporting layer ("HTL") is first deposited onto the ITO (indium tin oxide) coated glass substrate. For the device yielding 12% quantum efficiency, the HTL consisted of 30 nm (300 Å) of NPD. Onto the NPD a thin film of the organometallic doped into a host matrix is deposited to form an emitter layer. In the example, the emitter layer was CBP with 12% by weight bis(2-phenylbenzothiazole) iridium acetylacetonate (termed "BTIr"); the layer thickness was 30 nm (300 Å). A blocking layer is deposited onto the emitter layer. The blocking layer consisted of bathcuproine ("BCP"), and the thickness was 20 nm (200 Å). An electron transport layer is deposited onto the blocking layer. The electron transport layer consisted of Alq3 of thickness 20nm. The device is finished by depositing a Mg-Ag electrode onto the electron transporting layer. This was of thickness 100nm. All of the depositions were carried out at a vacuum less than 5 X 10⁻⁵ Torr. The devices were tested in air, without packaging.

When we apply a voltage between the cathode and the anode, holes are injected from ITO to NPD and transported by the NPD layer, while electrons are injected from MgAg to Alq and transported through Alq and BCP. Then holes and electrons are injected into EML and carrier recombination occurs in CBP, the excited states were formed, energy transfer to BTIr occurs and finally BTIr molecules are excited and decay radiatively.

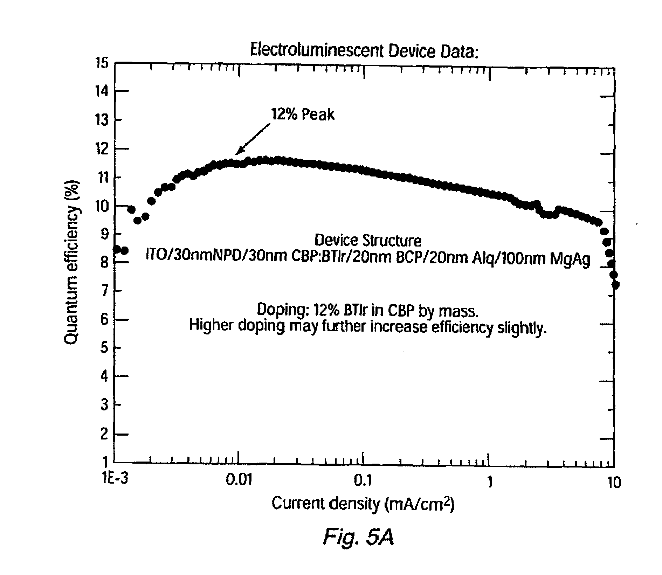

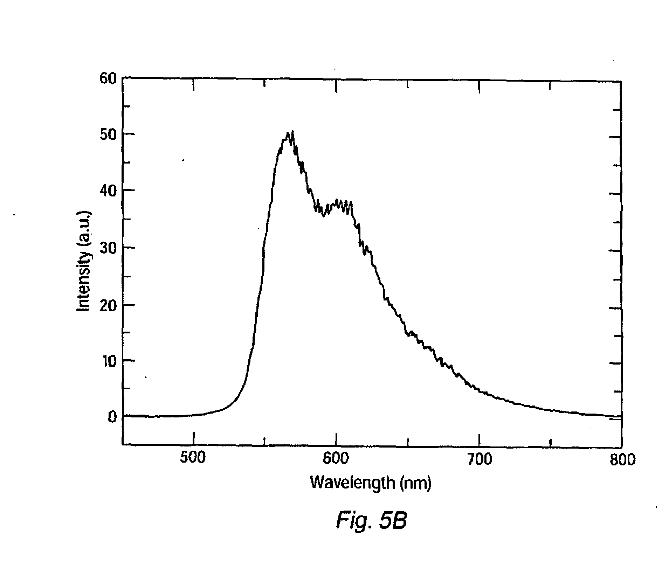

As illustrated in Figure 5, the quantum efficiency of this device is 12% at a current density of about 0.01 mA/cm2.

Pertinent terms are as follows:
ITO is a transparent conducting phase of indium tin oxide which functions as an anode.
ITO is a degenerate semiconductor formed by doping a wide band semiconductor. The carrier concentration of the ITO is in excess of 10¹⁹/cm³.
BCP is an exciton blocking and electron transport layer.
AIq3: electron injection layer.
Other hole transport layer materials could be used. For example, TPD, a hole transport layer, can be used.

BCP functions as an electron transport layer and as an exciton blocking layer, which layer has a thickness of about 10 nm (100 Å). BCP is 2,9-dimethyl-4,7-diphenyl-1,10-phenanthroline (also called bathocuproine) which has the formula

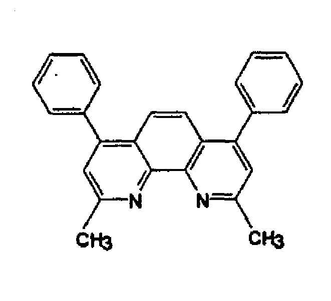

The Alq3, which functions as an electron injection/electron transport layer has the following formula.

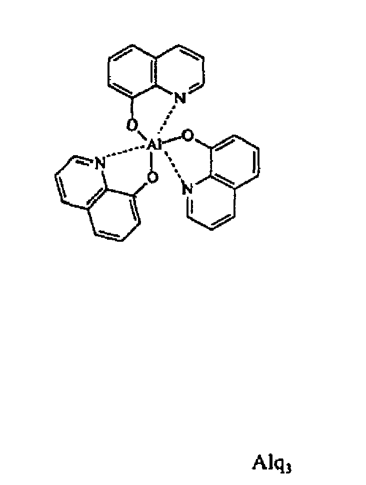

In general, the doping level is varied to establish the optimum doping level.

### V. B. 2. Incorporation of a fluorescent ligand into a phosphorescent complex

As noted above, fluorescent materials have certain advantages as emitters in devices. If the L ligand that is used in making the L₂MX (for example, M=Ir) complex has a high fluorescent quantum efficiency, it is possible to use the strong spin orbit coupling of the Ir metal to efficiently intersystem cross in and out of the triplet states of the ligands. The concept is that the Ir makes the L ligand an efficient phosphorescent center. Using this approach, it is possible to take any fluorescent dye and make an efficient phosphorescent molecule from it (that is, L fluorescent but L₂MX (M=Ir) phosphorescent).

As an example, we prepared a L₂IrX wherein L=coumarin and X=acac. We refer to this as coumarin-6 ["C6Ir"]. The complex gives intense orange emission, whereas coumarin by itself emits green. Both coumarin and C6Ir spectra are given in the figures.

Other fluorescent dyes would be expected to show similar spectral shifts. Since the number of fluorescent dyes that have been developed for dye lasers and other applications is quite large, we expect that this approach would lead to a wide range of phosphorescent materials.

One needs a fluorescent dye with suitable functionality such that it can be metallated by the metal (for example, iridium) to make a 5 or 6 membered metallocycle. All of the L ligands we have studied to date have sp2 hybridized carbons and heterocyclic N atoms in the ligands, such that one can form a five membered ring on reacting with Ir.

### V.B.3. Carrier trapping at X or L ligands

Potential degradation reactions, involving holes or electrons, can occur in the emitter layer. The resultant oxidation or reduction can alter the emitter, and degrade performance.

In order to get the maximum efficiency for phosphor doped OLEDs, it is important to control the holes or electrons which lead to undesirable oxidation or reduction reactions. One way to do this is to trap carriers (holes or electrons) at the phosphorescent dopant. It may be beneficial to trap the carrier at a position remote from the atoms or ligands responsible for the phosphorescence. The carrier that is thus remotely trapped could readily recombine with the opposite carrier either intramolecularly or with the carrier from an adjacent molecule.

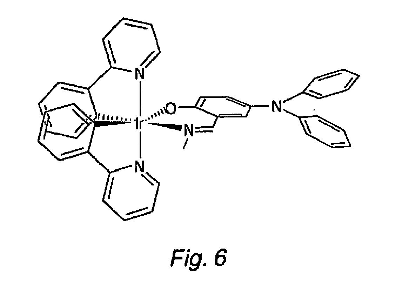

An example of a phosphor designed to trap holes is shown in Figure 6. The diarylamine group on the salicylanlide group is expected to have a HOMO level 200-300 mV above that of the Ir complex (based on electrochemical measurements), leading to the holes being trapped exclusively at the amine groups. Holes will be readily trapped at the amine, but the emission from this molecule will come from MLCT and intraligand transitions from the Ir(phenylpyridine) system. An electron trapped on this molecule will most likely be in one of the pyridyl ligands. Intramolecular recombinagtion will lead to the formation of an exciton, largely in the Ir(phenylpyridine) system. Since the trapping site is on the X ligand, which is typically not involved extensively in the luminescent process, the presence of the trapping site will not greatly affect the emission energy for the complex. Related molecules can be designed in which electron carriers are trapped remoted to the L₂Ir system.

### V.B.4. Color Tuning

As found in the IrL₃ system, the emission color is strongly affected by the L ligand. This is consistent with the emission involving either MLCT or intraligand transitions. In all of the cases that we have been able to make both the tris complex (i.e., IrL₃) and the L₂IrX complex, the emission spectra are very similar. For example Ir(ppy)₃ and (ppy)₂Ir(acac) (acronym = **PPIr)** give strong green emission with a λₘₐₓ of 510 nm. A similar trend is seen in comparing Ir(BQ), and Ir(thpy)₃ to their L₂Ir(acac) derivatives, i.e. , in some cases, no significant shift in emission between the two complexes.

However, in other cases, the choice of X ligand affects both the energy of emission and efficiency. Acac and salicylanilide L₂IrX complexes give very similar spectra. The picolinic acid derivatives that we have prepared thus far show a small blue shift (15 nm) in their emission spectra relative to the acac and salicylanilide complexes of the same ligands. This can be seen in the spectra for **BTIr, BTIrsd and BTIrpic.** In all three of these complexes we expect that the emission becomes principally form MLCT and Intra-L transitions and the picolinic acid ligands are changing the energies of the metal orbitals and thus affecting the MLCT bands.

If an X ligand is used whose triplet levels fall lower in energy than the "L₂Ir" framework, emission from the X ligand can be observed. This is the case for the **BTIRQ** complex. In this complex the emission intensity is very weak and centered at 650 nm. This was surprising since the emission for the BT ligand based systems are all near 550 nm. The emission in this case is almost completely form Q based transitions. The phosphorescence spectra for heavy metal quinolates (e.g. IrQ₃ or PtQ₂) are centered at 650 run. The complexes themselves emit with very low efficiency, < 0.01. Both the energy and efficiency of the L₂IrQ material is consistent "X" based emission. If the emission form the X ligand or the "IrX" system were efficient this could have been a good red emitter. It is important to note that while all of the examples list here are strong "L" emitters, this does not preclude a good phosphor from being formed from "X" based emission.

The wrong choice of X ligand can also severally quench the emission from L₂IrX complexes. Both hexafluoro-acae and diphenyl-acac complexes give either very weak emission of no emission at all when used as the X ligand is L₂IrX complexes. The reasons why these ligands quench emission so strong are not at all clear, one of these ligands is more electron withdrawing than acac and the other more electron donating. We give the spectrum for **BQIrFA** in the figures. The emission spectrum for this complex is slightly shifted from **BQIr,** as expected for the much stronger electron withdrawing nature of the hexafluoroacac ligand. The emission intensity from **BQIrFA** is at least 2 orders of magnitude weaker than **BQIr.** We have not explored the complexes of these ligands due this severe quenching problem.

### V. C. Other molecular depictions

CBP was used in the device described herein. The invention will work with other hole-transporting molecules known by one of ordinary skill to work in hole transporting layers of OLEDs.

Specifically, the invention will work with other molecules comprising a carbazole functionality, or an analogous aryl amine functionality.

### V.D. Uses of device

The OLED of the present invention may be used in substantially any type of device which is comprised of an OLED, for example, in OLEDs that are incorporated into a larger display, a vehicle, a computer, a television, a printer, a large area wall, theater or stadium screen, a billboard or a sign.

The present invention as disclosed herein may be used in conjunction with copending applications: "High Reliability, High Efficiency, Integratable Organic Light Emitting Devices and Methods of Producing Same", Serial No. 08/774,119 (filed December 23, 1996). "Novel Materials for Multicolor Light Emitting Diodes", Serial No 08/850,264 (filed May 2, 1997); "Electron Transporting and Light Emitting Layers Based on Organic Free Radicals", Serial No. 08/774,120 (filed December 23, 1996)(issued as US 5,811,833 on Sept. 22, 1998); "Multicolor Display Devices", Serial No. 08/712,333 (filed December 23, 1996); "Red-Emitting Organic Light Emitting Devices (OLED's)". Serial No. 08/774,087 (filed December 23, 1996)(allowed); "Driving Circuit For Stacked Organic Light Emitting Devices", Serial No. 08/792,050 (filed February 3, 1997)(issued as US 5,757,139 on May 26, 1998); "High Efficiency Organic Light Emitting Device Structures", Serial No. 08/772,332 (filed December 23, 1996)(issued as US 5,834,893 on Nov. 10,1998); "Vacuum Deposited, Non-Polymeric Flexible Organic Light Emitting Devices". Serial No. 08/789,319 (filed January 23, 1997)(issued as US 5,844,363 on Dec. 1, 1998); "Displays Having Mesa Pixel Configuration", Serial No. 08/794,595 (filed February 3, 1997); "Stacked Organic Light Emitting Devices", Serial No. 08/792,046 (filed February 3, 1997)(issued as US 5,917,280 on June 29.1999); "High Contrast Transparent Organic Light Emitting Devices", Serial No. 08/792,046 (filed February 3, 1997); "High Contrast Transparent Organic Light Emitting Device Display", Serial No. 08/821,380 (filed March 20, 1997); "Organic Light Emitting Devices Containing A Metal Complex of 5-Hydroxy-Quinoxaline as A Host Material", Serial No. 08/838,099 (filed April 15, 1997)(issued as US 5,861,219 on Jan. 19,1999); "Light Emitting Devices Having High Brightness", Serial No. 08/844,353 (filed April 18, 1997); "Organic Semiconductor Laser", Serial No. 08/859,468 (filed May 19,1997); "Saturated Full Color Stacked Organic Light Emitting Devices", Serial No. 08/858,994 (filed on May 20,1997)(issued as US 5,932,895 on Aug. 3, 1999); "Plasma Treatment of Conductive Layers", PCT/US97/10252, (filed June 12,1997); "Novel Materials for Multicolor Light Emitting Diodes", Serial No. 08/814,976, (filed Mar. 11, 1997); "Novel Materials for Multicolor Light Emitting Diodes", Serial No. 08/771,815, (filed Dec. 23, 1996); "Patterning of Thin Films for the Fabrication of Organic Multi-color Displays", PCT/US97/10289, (filed June 12, 1997), and "Double Heterostructure Infrared and Vertical Cavity Surface Emitting Organic Lasers", PCT/US98/09480 filed May 8, 1998; US 5,874,803 issued Feb. 23, 1999; US 5,707,745 issued Jan. 13, 1998; US 5,703,436 issued Dec. 30,1997; and US 5,757,026 issued May 26, 1998 each copending application or patent being incorporated herein by reference in its entirety.

An invention is further described in terms of following numbered paragraphs:
1. An organic light emitting device comprising an emitter layer comprising a molecule of the formula L₂MX, wherein L and X are inequivalent, bidentate ligands and M is a metal which forms octahedral complexes, and wherein the emitter layer produces an emission which has a maximum at a certain wavelength λₘₐₓ.
2. An organic light emitting device comprising an emitter layer comprising a host and a dopant wherein the dopant comprises a molecule of the formula L₂MX, wherein L and X are inequivalent, bidentate ligands and M is a metal which forms octahedral complexes.
3. The device of paragraph 1 wherein L is selected from the group consisting of 2-(1-naphthyl)benzoxazole), (2-phenylbenzoxazole), (2-phenylbenzothiazole), (2-phenylbenzothiazole), (7,8-benzoquinoline), coumarin, (thienylpyridine), phenylpyridine, benzothienylpyridine, 3-methoxy-2-phenylpyridine, thienylpyridine, and tolylpyridine; and X is selected from the group consisting of acetylacetonate ("acac"), hexafluoroacetylacetonate, salicylidene, picolinate, and 8-hydroxyquinolinate.
4. The device of paragraph 1 wherein M is iridium.
5. The device of paragraph 1 wherein M is iridium and wherein L is selected from the group consisting of 2-(1-naphthyl)benzoxazole), (2-phenylbenzoxazole), (2-phenylbenzothiazole), (2-phenylbenzothiazole), (7,8-benzoquinoline), coumarin, (thienylpyridine), phenylpyridine, benzothienylpyridine, 3-methoxy-2-phenylpyridine, thienylpyridine, and tolylpyridine; and X is selected from the group consisting of acetylacetonate ("acac"), hexafluoroacetylacetonate, salicylidene, picolinate, and 8-hydroxyquinolinate.
6. The device of paragraph 1 wherein L is a fluorescent and L₂MX is phosphorescent.
7. The device of paragraph 2 wherein the organic molecule of the host is selected from the group consisting of 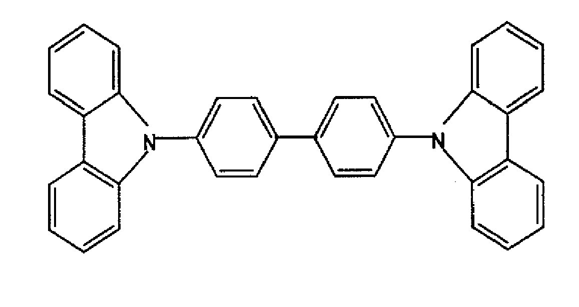 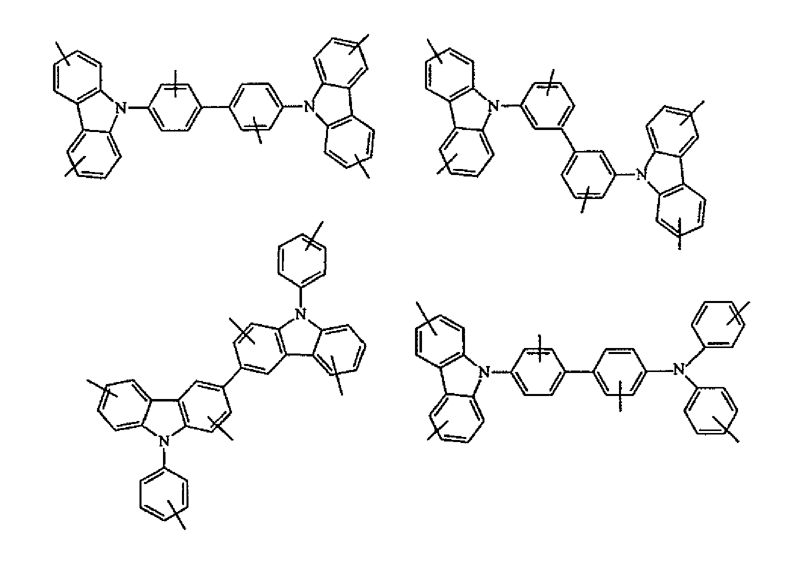 wherein the notation of the line segment drawn through the aromatic ring denotes optional substitution at any carbon in that ring by alkyl or aryl.
8. The organic light emitting device of paragraph 2 comprising an emitter layer comprising a molecule comprising a moiety L₂M, wherein L is a monoanionic bidentate ligand coordinated to M through an sp² carbon and a heteroatom and M is a metal which forms octahedral complexes, and wherein the heteroatoms of the two L ligands are in a trans configuration.
9. The device of paragraph 1 wherein the metal is selected from the group consisting of osmium, iridium and platinum.
10. The device of paragraph 1 wherein X functions to trap electrons or holes.
11. The device of paragraph 1 wherein X is selected such that there is at least a 15 nm difference in λₘₐₓ between L₂MX and L₃M.
12. The device of paragraph 1 wherein L₂MX is made from L₂M(µ-Cl)₂ML₂.
13. The device of paragraph 1 being incorporated into a larger display, a vehicle, a computer, a television, a printer, a large area wall, theater or stadium screen, a billboard or a sign.
14. An organic light emitting device comprising an emitter layer comprising a molecule of the formula L L' L" M, wherein L, L', and L" are inequivalent bidentate ligands and M is a metal which forms octahedral complexes, and wherein L L' L" M is phosphorescent.
15. An organic light emitting device comprising an emitter layer comprising a molecule comprising an L2M moiety, wherein L is a bidentate ligand comprising nitrogen which coordinates to M and M is a metal which forms octahedral complexes, and wherein the nitrogens of the two L-M bonds are in a trans configuration to one another.
16. A composition of formula L L' L" M, wherein L, L', and L" are bidentate ligands which coordinate to M and M is a metal selected from the third row of the transition metal group of the periodic table which forms an octahedral complex with L, L', and L".
17. The composition of formula L L' L" M of paragraph 15, wherein L L' L" M electroluminescences via a phosphorescent mechanism.
18. The composition of paragraph 15 wherein each of L, L', and L" comprises one nitrogen which coordinates to M and the nitrogens are in a meridianal arrangement.
19. The composition of paragraph 15 wherein L and L' are bidentate monoanionic ligands comprising nitrogen which coordinates to M and L" is a bidentate monoanionic ligand.
20. The composition of paragraph 15 wherein L and L' are equivalent and are monoanionic bidentate ligands which coordinate to M via an sp² hybridized carbon and a heteroatom and L" is a monoanionic bidentate ligand.
21. The composition of paragraph 19 selected from the group consisting of Ir(3-MeOppy)₃, tpylrsd, thpylrsd, btlrsd, BQlr, BQIrFA, THPIr, PPlr, BTPlr, tpylr, C6Ir, PZlrp, BONlr, BTIr, BOlr, BTIrQ, BTIrP, and BOIrP.
22. An organic light emitting device comprising an emitter layer comprising a molecule comprising a moiety L₂M, wherein L is a monoanionic bidentate ligand coordinated to M through an sp² carbon and a heteroatom and M is a metal which forms octahedral complexes, and wherein the heteroatoms of the two L ligands are in a trans configuration.
23. The method of making the composition of paragraph 19 comprising the step of combining a bridged dimer of formula L₂M(µ-Cl)₂ML₂ with a Bronsted acid XH to make an organometallic complex of formula L₂MX wherein L and X are monoanionic, bidentate ligands.

## Claims

1. A complex of formula LL'L"M, LL'MX, LMXX', or L₃M, wherein
L, L', L", X, and X' are inequivalent, bidentate ligands,
M is a metal that forms octahedral complexes, and
L, L', and L" are monoanionic bidentate ligands coordinated to M through an sp² hybridized carbon and a heteroatom.

2. The complex of claim 1, wherein the heteroatom of the L, L' and L" ligands is nitrogen.

3. The complex of claim 1 or 2, wherein the ring comprising the metal M, the sp² hybridized carbon and the heteroatom consists of five atoms.

4. The complex of claim 1 or 2, wherein the ring comprising the metal M, the sp² hybridized carbon and the heteroatom consists of six atoms.

5. The complex of claim 1, 2, 3 or 4, wherein M is selected from the group consisting of the transition metals of the third row of the transition series of the periodic table.

6. The complex of claim 1, 2, 3 or 4, wherein M is Ir.

7. The complex of claim 1, 2, 3 or 4, wherein M is Pt.

8. The complex of any one of the previous claims, wherein the L, L' and L" ligands are independently selected from the group consisting of 2-(1-naphthyl)benzoxazole, 2-phenylbenzoxazole, 2-phenylbenzothiazole, 7,8-benzoquinoline, coumarin, thienylpyridine, phenylpyridine, benzothienylpyridine, 3-methoxy-2-phenylpyridine, and tolylpyridine.

9. The complex of any one of the claims 1 through 7, wherein the L, L' and L" ligands are independently selected from the group consisting of substituted and unsubstituted arylquinolines.

10. The complex of any one of the previous claims, wherein the X and X' ligands are independently selected from the group consisting of an acetylacetonate, a hexafluoroacetylacetonate, a salicylidene, a picolinate, a 8-hydroxyquinolinate, amino acids, salicylaldehydes, and iminoacetonates.

11. An organic light emitting device comprising a complex of any one of the previous claims.

12. The organic light emitting device of claim 11, further comprising an emissive layer wherein the emissive layer comprises a host and a dopant, and wherein the dopant comprises the complex of any one of the previous claims.

13. The organic light emitting device of claim 11 or 12, wherein the organic light emitting device is incorporated into a system selected from the group of systems consisting of a vehicle, a computer, a television, a printer, a large area wall, a billboard, a stadium screen, a theater screen and a sign.

14. Use of the complex of any one of the claims 1 through 10 in an organic light emitting device.

15. Use of the complex of any one of the claims 1 through 10 in an emissive layer of an organic light emitting device.
